(19) Europäisches Patentamt
European Patent Office
Office européen des brevets

(11) EP 0 774 160 B1

## (12) EUROPEAN PATENT SPECIFICATION

(45) Date of publication and mention
of the grant of the patent:
**06.10.1999 Bulletin 1999/40**

(21) Application number: **95927053.9**

(22) Date of filing: **31.07.1995**

(51) Int Cl.6: **H01J 49/02**

(86) International application number:
**PCT/IB95/00656**

(87) International publication number:
**WO 96/04676 (15.02.1996 Gazette 1996/08)**

(54) **MASS SPECTROMETER FOR MACROMOLECULES WITH CRYOGENIC PARTICLE DETECTORS**

MASSENSPEKTROMETER FÜR MACROMOLEKÜLE MIT KRYOGENISCHEN TEILCHENDETEKTOREN

SPECTROMETRE DE MASSE POUR MACROMOLECULES DOTE DE DETECTEURS DE PARTICULES CRYOGENIQUES

(84) Designated Contracting States:
**AT BE CH DE DK ES FR GB GR IE IT LI LU MC NL PT SE**

(30) Priority: **03.08.1994 CH 242494**
**11.05.1995 US 438707**

(43) Date of publication of application:
**21.05.1997 Bulletin 1997/21**

(73) Proprietor: **TWERENBOLD, Damian**
**2017 Boundry (CH)**

(72) Inventor: **TWERENBOLD, Damian**
**2017 Boundry (CH)**

(74) Representative:
**Smulders, Theodorus A.H.J., Ir. et al**
**Vereenigde Octrooibureaux**
**Nieuwe Parklaan 97**
**2587 BN 's-Gravenhage (NL)**

(56) References cited:
**EP-A- 0 360 676**

- **NUCLEAR INSTRUMENTS & METHODS IN PHYSICS RESEARCH, SECTION - A: ACCELERATORS, SPECTROMETERS, DETECTORS AND ASSOCIATED EQUIPMENT, vol. A294, 1990 AMSTERDAM NL, pages 259-267, Y. DE COULON, D. TWERENBOLD ET AL. 'CORRELATION MESUREMENTS OF IONIZING RADIATION INDUCED PHONONS IN SILICON USING SUPERCONDUCTING TUNNELING JUNCTIONS' cited in the application**
- **REVIEW OF SCIENTIFIC INSTRUMENTS, vol. 64, no. 3, March 1993 NEW YORK US, pages 737-740, XP 000355662 JEFFERTS S R ET AL 'SUPERCONDUCTING RESONATOR AND A CRYOGENIC GAAS FIELD-EFFECT TRANSISTOR AMPLIFIER AS A SINGLE-ION DETECTION SYSTEM'**

## Description

This invention relates to a mass spectrometer for macromolecules according to the preamble of claim 1. Such a mass spectrometer is known from EP-A-0,360,676, in which the molecules are detected by means of an ionizing particle detector comprising an electron multiplier.

In prior art mass spectrometry, various techniques have successfully been established to volatilize and ionize biological macromolecules: Fast Atom Bombardment (FAB), (1), Electro Spray Ionization (ESI), (2, 3) and Matrix Assisted Laser Desorption/Ionization (MALDI) (4, 5, 6, 7). In the MALDI method, the macromolecules are embedded with low concentration in a matrix of material with high photon absorption. When illuminated with high intensity laser light, the matrix heats up rapidly and evaporates into a plasma. During evaporation momentum is transferred to the macromolecules which are subsequently ionized in the plasma. Because the matrix plasma cools rapidly, most macromolecules remain intact. In prior art mass spectrometers the masses of those ionized macromolecules are determined with the time-of-flight (TOF) method (3, 7, 8), with the Fourier Transform Ion Cyclotron Resonance (FT-ICR) method (2, 5, 6) or with the single or multi quadrupole mass filter method (9, 10).

The disadvantage of prior art ionizing particle detectors used in mass spectrometers for macromolecules is the strong decrease of ionization efficiency for massive macromolecules owing to their decreasing particle velocites (11, 12). In state of the art detectors for mass spectrometry, the accelerated macromolecule emits an electron on impact with the detector which is subsequently multiplied by electron multiplier techniques. The efficiency to emit said first electron depends on the velocity of the impacting particle (11) which for a massive macromolecule is small. This lack of detector efficiency can be compensated for in prior art mass spectrometers by increasing the flux of macromolecules, however by the expense of decreasing the overall system sensitivity. Generally, in prior art mass spectrometers the detection of macromolecules with masses larger than typically 50,000 amu is inefficient. With the FT-ICR technique much larger masses can be detected, however, at the expense of large integration times of the order of one second, excluding applications where high throughput is required.

Mass spectrometry is used in biology for protein sequencing (9, 13) and protein identification (10) by measuring the mass distribution of protein-fragments. It is also considered to be a promising technique to increase the speed and to reduce the cost in DNA-sequencing (2, 3, 5, 6, 7, 8, 14). The standard DNA-sequencing procedure is to separate an aliquot of DNA-fragments, prepared according to the Maxam-Gilbert and Sanger strategy, using the pulsed gel-electrophoresis technique (15, 16). In this technique, the DNA-fragments are separated by their lengths according to their migration properties in a gel to which an electrical field is applied. The spatially separated bands of DNA-fragments are conventionally recorded by auto-radiography and fluorescent techniques.

The disadvantage of said gel-electrophoresis technique is the slow sequencing rate and the poor mass resolution for very large DNA-fragments. The disadvantage of prior art mass spectrometers for high rate DNA-sequencing is the low sensitivity of ionizing detectors for DNA-fragments consisting of more than 100 bases, making inaccessible the increase in DNA-sequencing rate which is possible with mass spectrometry. Accordingly, it is an object of this invention to provide a novel mass spectrometer for massive macromolecules.

It is a still further object of the invention to provide novel apparatus for measuring the masses of macromolecules with a detection efficiency independent of mass, i.e. allowing also the measurement of macromolecules with very high mass.

It is a still further object of the invention to provide novel apparatus for measuring the masses of macromolecules in the single particle counting mode.

It is a still further object of the invention to provide novel apparatus for measuring the masses of macromolecules with a detector system having both high temporal and high spatial resolution.

It is a still further object of the invention to provide novel apparatus for high throughput and high sensitivity base sequencing of short and long DNA-fragments.

It is a still further object of the invention to provide novel apparatus for high throughput and high sensitivity amino acid sequencing of small and large proteins.

It is a still further object of the invention to provide novel apparatus for high throughput and high sensitivity identification of small and large proteins.

It is a still further object of the invention to provide novel apparatus for high throughput and high sensitivity identification of small and large polymers.

Those objects are achieved by using phonon sensitive cryogenic particle detectors. In cryogenic particle detectors, the absorbed kinetic energy of the impacting accelerated macromolecules is converted into phonons (i.e. vibrations of the solid state lattice of the detector) which are converted into an electronic signal by phonon sensors. Said phonon sensors are sensitive only at cryogenic temperatures (i.e. temperatures less than a few Kelvin) where the background of thermal phonons is negligable.

Therefore, the mass spectrometer for macromolecules according to the present invention is characterized in that the detector system comprises a phonon sensitive cryogenic particle detector comprising one or more absorbers and one or more phonon sensors for detecting the time of impact and/or the position of impact of said accelerated macromolecules on the cryogenic detector; whereby the absorbed kinetic energy of said impacting macromolecules is converted into phonons excited in said absorber and whereby said phonon sensors convert said phonons into an electrical signal; a preamplifier system for converting said electronics signal of said cryogenic particle detector into a low impedance signal for further data processing; and a cryostat with a cold finger to which said cryogenic particle detector is thermally connected.

In accordance with the above and further objectives of the invention, one embodiment of apparatus is a time-of-flight mass spectrometer where the macromolecules are separated by their mass dependent velocities which is proportional to $\sqrt{m}$, where $m$ denotes the mass of the macromolecule. For the same emission time, heavier macromolecules arrive later at the position of the detector than corresponding lighter macromolecules. Said cryogenic particle detectors determine the arrival time of a macromolecule in the single particle counting mode with a sensitivity independent of mass, as it is the absorbed kinetic energy of the macromolecule which determines the efficiency of said detector, and not the velocity. The kinetic energy is for all said accelerated macromolecules the same.

In another embodiment, single or multiple quadrupole mass spectrometers separate and analyse the masses of macromolecules by quadrupole mass filters where cryogenic particle detectors measure the merging macromolecules. Very low quantities of macromolecules are required in this embodiment because of the single particle counting mode of said cryogenic particle detector.

In another embodiment, macromolecules are spatially separated by a stationary magnetic field according to their mass-to-charge ratio, subsequentially accelerated and then detected by spatial resolving cryogenic particle detectors.

In another embodiment, macromolecules are desorbed in a pulsed time sequence, spatially separated by a stationary magnetic field according to their mass-to-charge ratio, subsequentialy accelerated and then detected by spatial and temporal resolving cryogenic particle detectors. From the spatial and temporal information of the macromolecule event, and the known emission structure, the emission time of the macromolecule can be reconstructed and the mass determined from the time-of-flight. This leads to a parallel operating time-of-flight mass spectrometer with high sensitivity and throughput.

In accordance with the above and further objectives of the invention, one embodiment of said cryogenic particle detector is using crystal substrates as absorbers and superconducting tunneling junctions operated in the Giaever-mode as said phonon sensors.

In another embodiment of said cryogenic particle detector, the Giaever-type superconducting tunneling junctions are used both as absorbers and phonon sensors.

In another embodiment of said cryogenic particle detector, the phonon sensitivity of said Giaever-type superconducting tunneling junctions is enhanced by depositing said superconducting tunneling junctions on top of large area superconducting films which have a superconducting energy gap larger than the corresponding films of said superconducting tunneling junctions in order to use the quasiparticle trapping effect.

In another embodiment of said cryogenic particle detector, the crystal absorber is covered with superconducting transition edge phonon sensors.

In another embodiment of said cryogenic particle detector, microcalorimeters are used with consist of crystal absorber of low heat capacitance and high sensitivity thermistors as phonon sensors.

In another embodiment of said cryogenic particle detector, microcalorimeters are used with consist of crystal absorber of low heat capacitance and high sensitivity kinetic conductance thermometers as phonon sensors.

In another embodiment of said cryogenic particle detector, microcalorimeters are used where the crystal absorber and the thermal phonon sensor are identical.

In another embodiment of said cryogenic particle detector, the crystal absorber is covered with superconducting granules in the superheated phase which act as phonon sensors.

In another embodiment of said cryogenic particle detector, superconducting granules in the superheated phase are used both as absorbers and phonon sensors.

From the above summary, it can be understood that mass spectrometer of this invention has several advantages; (1) it allows the mass determination of a macromolecule with a sensitivity independent of the mass of the macromolecule, i.e. also for very massive macromolecules; (2) it allows the mass determination of a macromolecule in the single particle counting mode, i.e. enabling very high sensitivity; (3) the thin film technology for producing said phonon sensors allows for cryogenic detector array with high spatial resolution, i.e. high throughput is possible by spatially splitting the macromolecule beam and performing parallel measurements in time; (4) the sensitivity and throughput of DNA-sequencing can be improved by several orders of magnitude; (5) the sensitivity and throughput of protein- sequencing can be improved by several orders of magnitude; (6) the sensitivity and throughput of protein-identification can be improved by several orders of magnitude; (7) the sensitivity and throughput of polymer- identification can be improved by several orders of magnitude.

SUMMARY OF THE DRAWINGS

The above noted and other features of the invention will be understood from the following detailed description when considered with reference to the accompaning drawings in which:

FIG. **1** is a schematic of an embodiment of a phonon sensitive cryogenic detector;

FIG. **2** shows a schematic of the electronic circuitry of the embodiment shown in FIG. 1;

FIG. **3** is a schematic of an embodiment of the invention using a single channel cryogenic detector as detector in a MALDI-TOF mass spectrometer;

FIG. **4** is a schematic showing an embodiment of the mechanical shutter for the MALDI-TOF shown in FIG. **3**;

FIG. **5** illustrates the timing of the laser trigger and the shutter shown in FIG. **4**;

FIG. **6** illustrates the timing of events for three macromolecule masses for the embodiment shown in FIG. **3** and FIG. **4**;

FIG. **7** is a schematic of an embodiment of a spatial resolving cryogenic detector array, where the macromolecules are absorbed directly in the phonon sensor;

FIG. **8** is a schematic of an alternative embodiment of a spatial resolving cryogenic detector array, where the macromolecules are absorbed in the substrate and are sensed indirectly in the phonon sensor;

FIG. **9** is a schematic of an alternative embodiment of FIG. **8** where the phonon sensors are superconducting tunneling junctions with quasiparticle trapping films;

FIG. **10** shows the calculated trajectories of macromolecules of selected masses in an alternative embodiment using cryogenic detector arrays;

FIG. **11** is a schematic of an embodiment of a mass spectrometer with macromolecule trajectories as shown in FIG. **10**, where mass separation of the macromolecules occurs spatially in a magnetic field and the macromolecules are detected by a cryogenic detector array;

FIG. **12** shows the results of a calculation illustrating the dependence of the spatial mass resolving power versus post-acceleration voltage in the embodiment of FIG. **11**;

FIG. **13** shows the results of a calculation illustrating the dependence of the spatial mass separation versus molecular weight in the embodiment of FIG. **11**;

FIG. **14** shows the results of a calculation illustrating the dependence of the mass separation resolution versus molecular weight in the embodiment of FIG. **11**;

FIG. **15** shows the results of a calculation illustrating the dependence of the required magnetic field versus the required pre-acceleration voltage for detecting macromolecules in the embodiment of FIG. **11** in the mass range according to FIG. **10**;

FIG. **16** shows the results of a calculation illustrating the dependence of the post-acceleration versus the required pre-acceleration voltage for detecting macromolecules in the embodiment of FIG. **11** in the mass range according to FIG. **10**;

FIG. **17** shows the results of a Monte-Carlo calculation illustrating the position resolution of an embodiment of FIG. **7**, FIG. **8** or FIG. **9** used in the embodiment of FIG. **11**;

FIG. **18** shows the results of a Monte-Carlo calculation illustrating the reconstructed mass resolution as derived from FIG. **17**;

FIG. **19** illustrates the pulsed emission operating mode of the embodiment shown in FIG. **11**;

FIG. **20** shows the results of a Monte-Carlo calculation illustrating the arrival time resolution of an embodiment of FIG. **7**, FIG. **8** or FIG. **9** used in the embodiment of FIG. **11**;

FIG. **21** shows the results of a Monte-Carlo calculation illustrating the reconstructed mass resolution as derived from FIG. **20**;

FIG. **22** shows the results of a calculation illustrating the dependence of the mass reconstruction efficiency versus the duty cycle in the pulsed emission operating mode as shown in FIG. **19** in the embodiment as shown in FIG. **11**;

FIG. **23** shows the results of a calculation illustrating the dependence of the mass resolution as derived from the spatial resolution of the cryogenic detector array versus the spatial resolution of the cryogenic detector array in the embodiment as shown in FIG. **11**;

FIG. **24** shows the results of a calculation illustrating the dependence of the mass resolution as derived from the time-of-flight when using the the pulsed emission operating mode as shown in FIG. **19** versus the emission pulse length in the embodiment as shown in FIG. **11**;

FIG. **25** is a schematic of an embodiment of a probe sample used for DNA-sequencing in the embodiment as shown in FIG. **11**;

## DETAILED DESCRIPTION

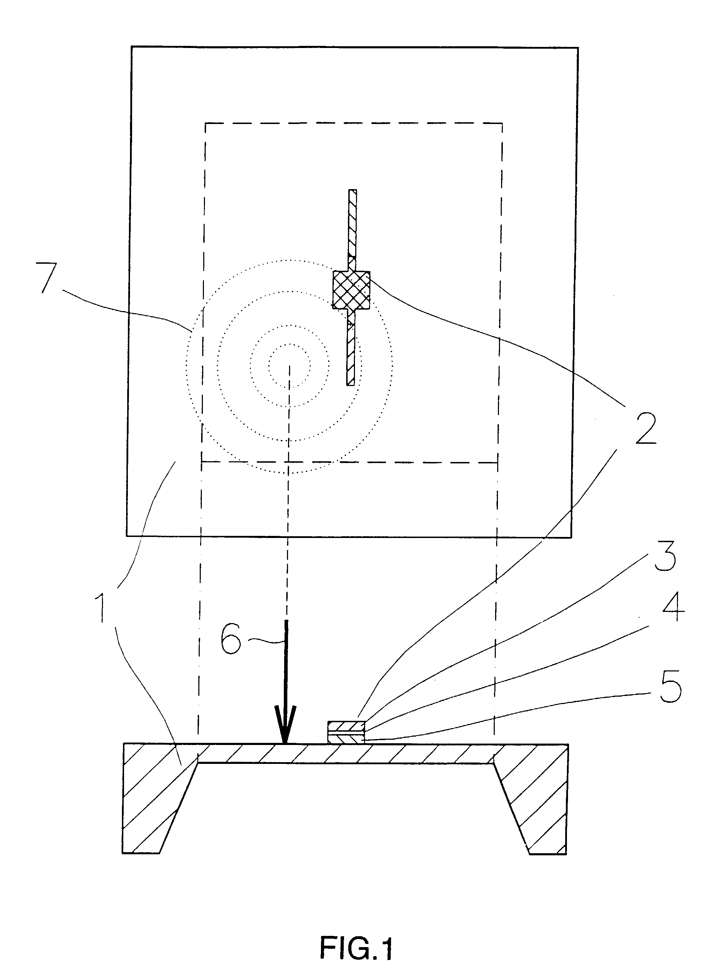

The schematic cryogenic particle detector shown in FIG. **1** consists of an absorber, indicated by the reference numeral **1**, onto which a phonon sensor **2** is deposited. In the embodiment shown in FIG. **1**, this phonon sensor is a superconducting tunneling junction consisting of a top film **3** of a few 100 nm separated by a thin oxide barrier 4 of a few nm and a bottom film **5** of a few 100 nm. Superconducting tunneling junctions are well established as $\alpha$-particle and x-ray detectors [17, 18, 19] and the physics is well understood [20]. Other embodiments of cryogenic phonon sensors are (see reference [19]): superconducting transition edge thermometers close to $T_c$, semiconducting thermistors, superconducting kinetic inductance thermometers and superheated superconducting granules and dots. Basically, the only requirement for the phonon sensor is to be sensitive to energy depositions of a few 10 keV and to have rise times not larger than 100 nsec. Cryogenic particle detectors operate at temperatures below a few Kelvin, where the background of thermally excited phonons is negligable. The operating principle of a phonon sensitive cryogenic particle detector for the mass spectrometry of macromolecules is the following (see FIG. **1**): a macromolecule **6** which has been accelerated to a kinetic energy of typically a few **10** keV by the electric field in the mass spectrometer produces phonons **7** which propagate through the absorber **1** and are eventually, converted in the phonon sensor **2** into an electric signal. The sensitivity of phonon sensitive cryogenic particle detectors to the absorption of ionizing particles with an energy of a few keV has been demonstrated by [21] and by other authors (see references in [19]). The novelty of this invention is the implementation of cryogenic particle detectors in a mass spectrometer for massive macromolecules. Cryogenic particle detectors, as shown in the embodiment of FIG. **1**, have the unique property, that they do not only detect ionizing particles, but that they are equally or more sensitive to the nonionizing direct transfer of kinetic energy to the lattice of the absorber. In said embodiment shown in FIG. **1**, the phonon collection efficiency is enhanced by etching down the substrate, e.g. single crystal silicon, to a thickness of a few 10 $\mu$m in order to localize the nonthermal phonon density in the vicinity of the phonon sensor.

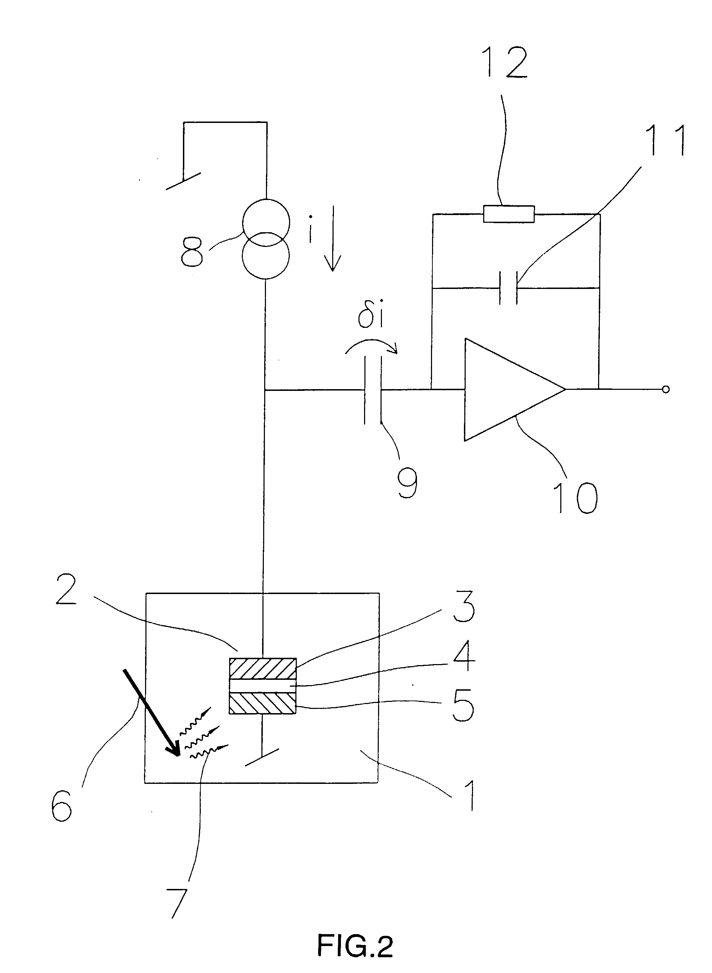

In FIG. **2** the electronic read circuitry is shown in the case of the embodiment of a superconducting tunneling junction as a phonon sensor: The phonons **7** produced by the absorption of a macromolecule **6** propagate through the absorber **1** of which some enter the superconducting films **3** and **5**. There, the phonons with energy larger than the Cooper pair binding energy 2Δ break Cooper pairs (the coherent electronic bound states in a superconductor) and produce excess quasiparticles (the electronic single excitation states in a superconductor). The two superconducting films **3** and **5** are at different energy potentials owing to the biasing current **i** which is provided by the current source **8**. A net current of excess quasiparticles then tunnels across the insulating barrier **4** (the tunneling of Cooper pairs, the DC Josephson current, is prohibited by a magnetic field applied parallel to the insulating barrier). As the phonons **7** and the corresponding excited excess quasiparticles decay on the time scale of a few $\mu$sec, the excess quasiparticle current δ**i** is of transient nature and will flow through the capacitor **9**. With a charge sensitive preamplifier consisting of a suitable operation amplifier **10**, a feed back capacitor **11** and a feed back resisitor **12** the excess quasiparticle current δ**i** is integrated and the integrated charge will be proportional to the number of phonons **7** absorbed in the phonon sensor **2**. Macromolecules can of course also be absorbed in the phonon sensor itself and produce directly a signal, which is an alternative embodiment of said cryogenic particle detector.

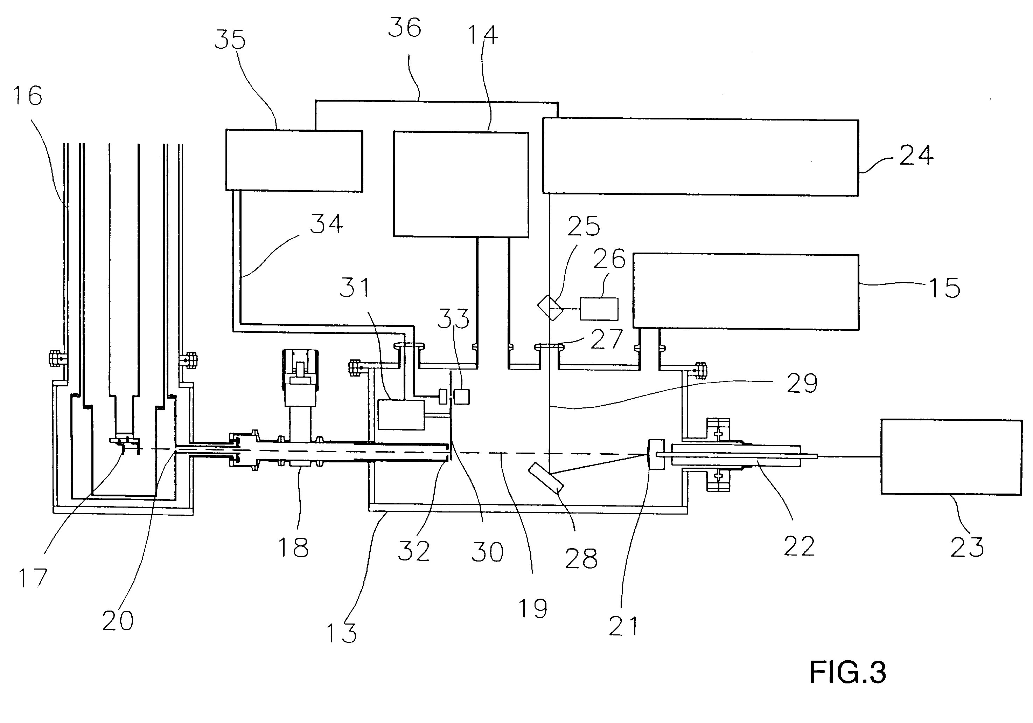

FIG. **3** shows a an embodiment of a mass spectrometer for macromolecules with cryogenic particle detectors using a setup generally referred to as a MALDI-TOF (matrix assisted laser desorption/ionization time of flight). A vacuum vessel **13** is evacuated by a turbo pump system **14** to a vacuum of about $10^{-5}$ mbar which is monitored by a vacuum measurement system **15**. A cryostat **16** with the cryogenic particle detector **17** attached to the cold finger is connected to the vacuum vessel **13** via a valve **18**. The beam of macromolecules **19** produced in the vacuum vessel **13** enters the cold area of the cryostat **16** by a series of small holes **20** in the cold shields of the cryostat. The beam of macromolecules **19** is produced by mounting the macromolecule sample **21** on a high voltage feed through **22** which is connected to a high voltage power supply **23** and by illuminating the sample with a laser beam **29** from a laser source **24**. The laser can be an UV-laser or an infrared laser. The laser beam **29** emerging from the laser source **24** is split in a beam splitter **25**: one part of the beam is used to measure the laser power in a power meter **26** and the other part enters the vacuum vessel **13** via the window **27**. In the vacuum vessel, the laser beam **29** is directed to the sample **21** via a mirror **28**. The probe consists of a light-sensitive matrix solution (e.g. sinapinic acid or $\alpha$-cyano-4-hydroxycinnamic acid [12]) into which the macromolecules have been diluted in ratios exceeding $10^4$:1. The laser power, typically a few mJ in a few nsec, is absorbed by the matrix, which explodes and turns into an electric plasma. The expanding matrix transfers momentum to the macromolecules which are thus volatilized and subsequently, charged by the plasma character of the expanding matrix. Owing to the electric field in the vacuum vessel produced by the high voltage on **22,** the macromolecules with the same charge as the high voltage potential will be accelerated towards the transfer tube **32** into the cryostat **16**, through the holes of the cooling shields **20** and finally onto the cryogenic detector **17**. The time difference of the laser trigger and the time of arrival signal of the cryogenic particle detector is a measure for the mass of the macromolecule. As the cryogenic particle detector is a very sensitive device, no light and no low mass debris from the matrix should hit the detector, which most probably would lead to a saturation of the detector and/or heating up of the cryostat. In order to prevent this, a mechanical shutter **30** is operated in front of the transfer tube **32**. In one embodiment of the shutter, a motor **31** turns a disk with a slit where a light emitting diode and light detection system **33** measures the position of the slit and an electronic control system **35** gives an appropriate trigger signal **36** to the laser **24**.

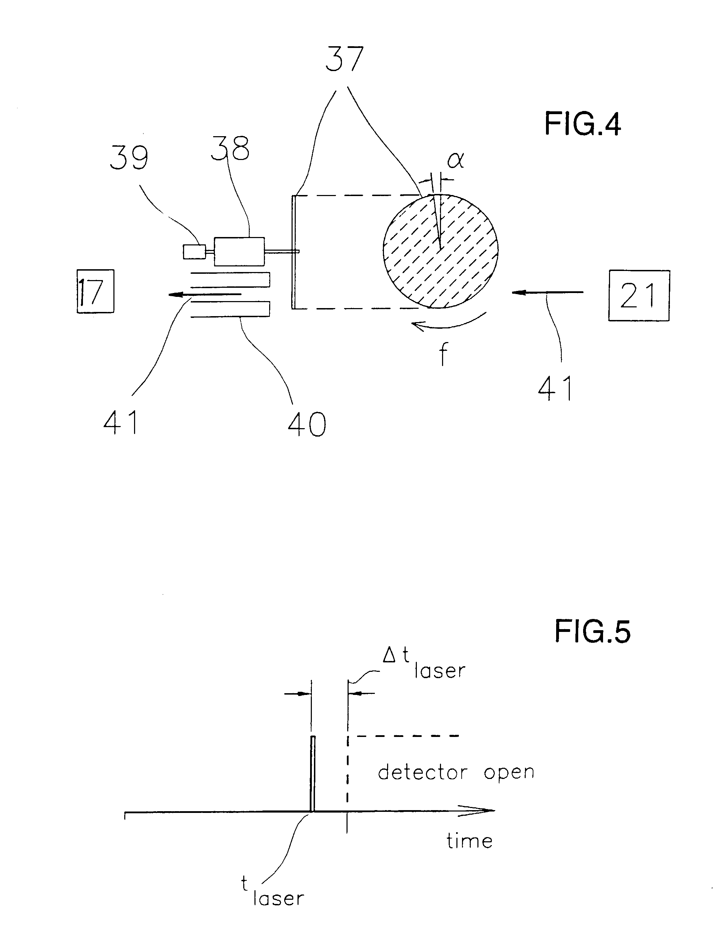

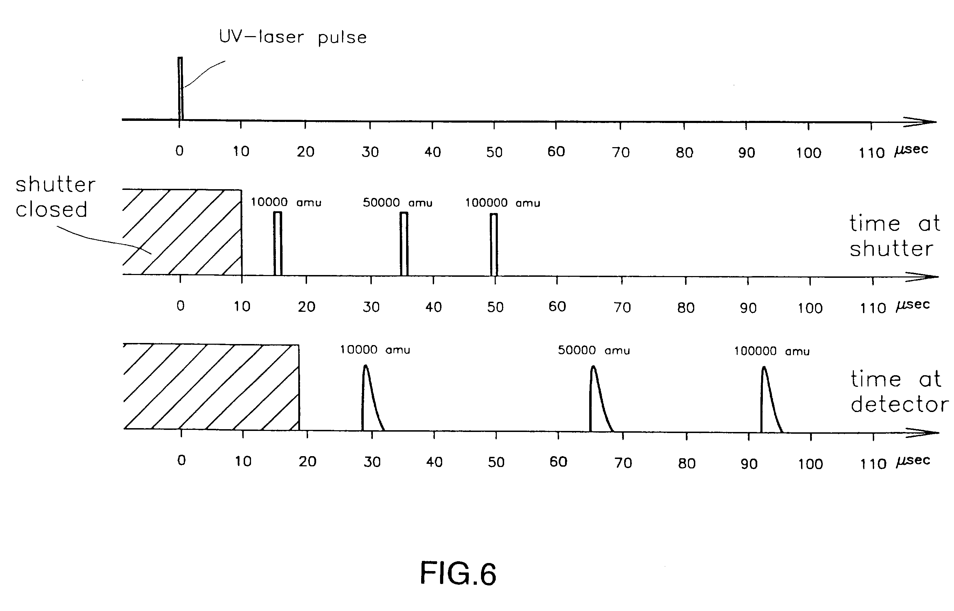

In FIG. **4**, a particular embodiment of this mechanical shutter is shown: a motor 38 turns a disk **37** with a slit of opening $\alpha$ (typically 5° to 10°) at a frequency f (typically between **50** and 100 Hz). The position of the rotating shaft is monitored by an incremental decoder **39**. Accelerated macromolecules **41** from the source **21** will enter the tube **40** connecting the vacuum vessel to the cryostat only when the slit of **37** is at the position of the tube. By suitable choice of the timing of the laser trigger, the beam **41** of macromolecule hitting the cryogenic particle detector **17** will consist only of macromolecules with masses larger than a certain cutoff value. As is shown in FIG. **5**, the electronic control system of the mechanical shutter will be such that a trigger signal $\mathbf{t}_{laser}$ will be transmitted to the laser a specified time interval $\Delta \mathbf{t}_{laser}$ prior to the opening of the shutter **37** at the position of the transfer tube **40**. The detector is then exposed to a specified mass range of macromolecules depending on the angle $\alpha$ of the opening of the slit and the rotating speed f of the shutter. In FIG. **6**, the arrival times of macromolecules with different masses is shown at the position of the rotating mechanical shutter and at the position of the detector for the embodiment shown in FIG. **3**. Because the kinetic energy is proportional to $mv^2$, where $m$ is the mass and $v$ the velocity of the macromolecule, the time-of-flight is proportional to $\sqrt{m}$. The shaded area at times prior to 10 $\mu sec$ in this example corresponds to the mass range of macromolecules which does not reach the detector.

In order to improve the throughput, another embodiment of the invention uses cryogenic detector arrays which resolve both the time of impact and the position of impact of the accelerated macromolecule on said cryogenic detector array. As many embodiments of cryogenic detectors use thin film deposition and lithography techniques, small scale structuring on the $\mu$m level will provide a high spatial resolution of the position of impact. In this preferred embodiment of the invention, macromolecules are separated spatially in a magnetic field by their mass/charge ratio and the mass is determined by the position of impact of the macromolecule on the cryogenic detector array. When, in another version of this embodiment, a pulsed emission technique is used, the mass spectrometer is a highly parallel time-of-flight mass spectrometer, allowing the simultaneous determination of a large range of masses in the short succession of a few $\mu$sec. This embodiment of the mass spectrometer will be discussed in detail below. First some embodiments of the cryogenic detector arrays will be presented.

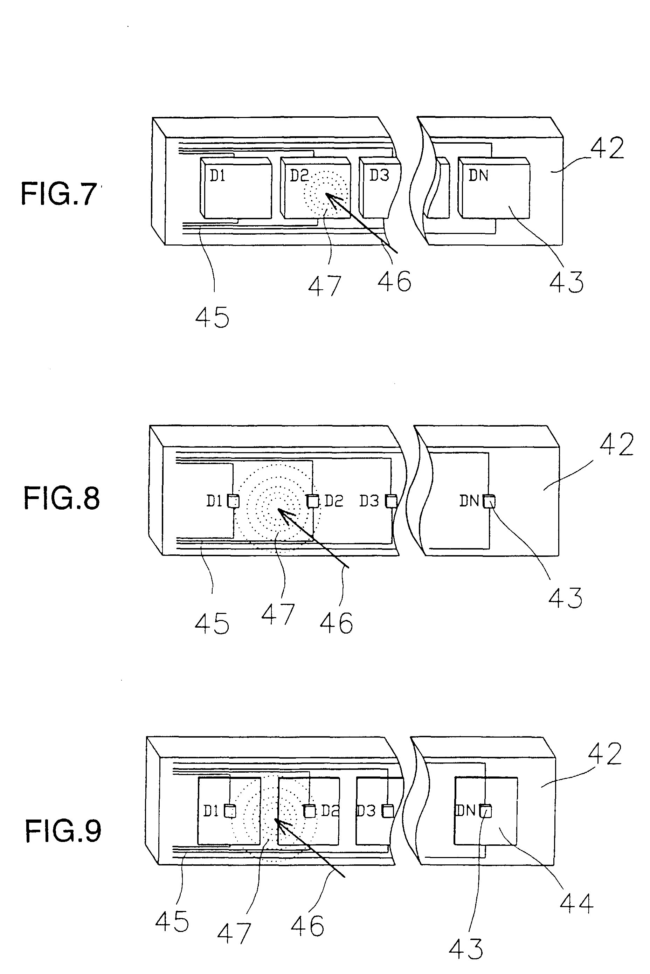

The cryogenic detector array used in this invention consists of a number N of phonon sensors **D1** ... **DN** with current leads **45** on a substrate **42** as shown in FIG. **7**, FIG. 8 and FIG. **9**. In the embodiment of a cryogenic detector array shown in FIG. **7**, the accelerated macromolecule **46** is absorbed directly in the phonon sensor **43** where phonons **47** are produced and are turned into an electronic signal directly. The advantage of this embodiment is the high efficiency of phonon-to-charge conversion and the fast timing signal owing to this direct process. However, phonon sensors with large area surface areas are required, which may be technologically, difficult to fabricate (at least in the case of superconducting tunneling junctions). An alternative in said embodiment of FIG. **7** would be a large number of phonon sensors with small areas which, however, would lead to a very large number of electronic channels **45**. Therefore, a preferable embodiment of a cryogenic detector array is shown in FIG. **8**; the accelerated macromolecule **46** is absorbed in the absorber and the produced phonons **47** propagate through the absorber and are converted into an electronic signal by the phonon sensors **43**. When pure single crystals are used as substrates, e.g. single crystal silicon, the phonons can propagate ballistically large distances and will be sensed than more than one phonon sensor. In said embodiment where superconducting tunneling junctions are used as phonon sensors, the electronic signal is proportional to the number of phonons absorbed and the pulse heigth is proportional to the superconducting tunneling junction's distance to the the point of interaction. Hence, a precise determination of this point of macromolecule absorption can be determined by calculating the centroid of the different pulse heights corresponding to the various junctions responding to the phonon pulse. An alternative embodiment of FIG. **8** is shown in FIG. **9** where an additional superconducting film **44** is deposited under the superconducting tunneling junction. The material of this superconducting film is chosen such that its superconducting energy gap $\Delta$ is larger than the corresponding gap of the junction, in order for the quasiparticle trapping effect to occur [22]. The quasiparticles produced by the phonons **47** in **44** will propagate via quasiparticle diffusion in **44** and ultimately be trapped in the lower film of the superconducting tunneling junction (see **5** of FIG. **1**). There they will tunnel through the oxid barrier and produce the detector signal as described above. The embodiment of FIG. **9** improves the phonon collection efficiency of FIG. **8**. Calculations presented below show that the requirements on spatial and temporal resolution of the CDA are technologically, reasonable: in the specific model of the embodiment of the invention presented in the following paragraph, a spatial resolution of $\delta x=0.1$ mm and a temporal resolution of $\delta t=100$ nsec turns out to be sufficient for achieving a mass resolution of 100 amu for a mass of 600000 amu. In those calculations, the cryogenic detector array is 10 cm in length and consists of 100 phonon sensors. Other embodiments of phonon sensors have been mentioned above in the discussion of FIG. **1**.

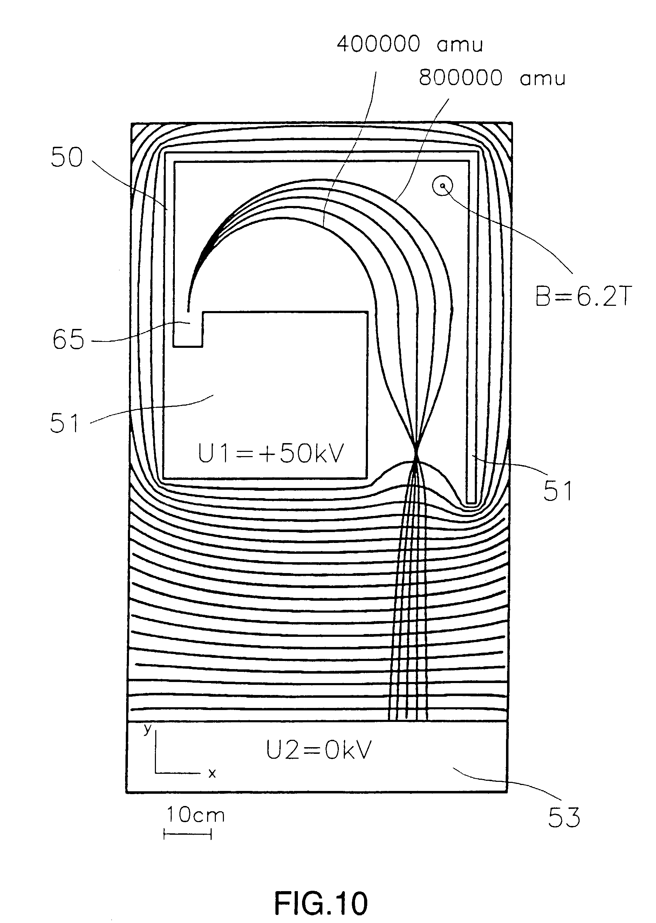

With the cryogenic detector arrays presented above, the preferred embodiment of this invention is a mass spectrometer with calculated macromolecule trajectories as shown in FIG. **10**: macromolecules are volatilized, ionized and pre-accelerated in **51**, then separated by their mass/charge ratios in a magnetic field **50**, subsequently accelerated electrostatically by the potential difference **U1 - U2** and detected by the cryogenic detector array **53**. There, both the position of impact and the time of impact of the macromolecules are determined by in the single particle counting mode. Again, it should be mentioned that one of the major improvements of this invention is the sensitivity, of the cryogenic detector also for high macromolecule masses. In the following, a specific design of the preferred embodiment is presented, and the results of calculations of the corresponding design are discussed. According to those calculations masses of up to $10^6$ amu could be measured with a resolution of 100 amu. In addition, the mass spectrometer can be operated in a quasi-continuos mode with a duty cycle of 10%. Because of the single counting detection mode of the cryogenic detectors, only a small amount of macromolecules would be required, typically considerably, less than a femtomol.

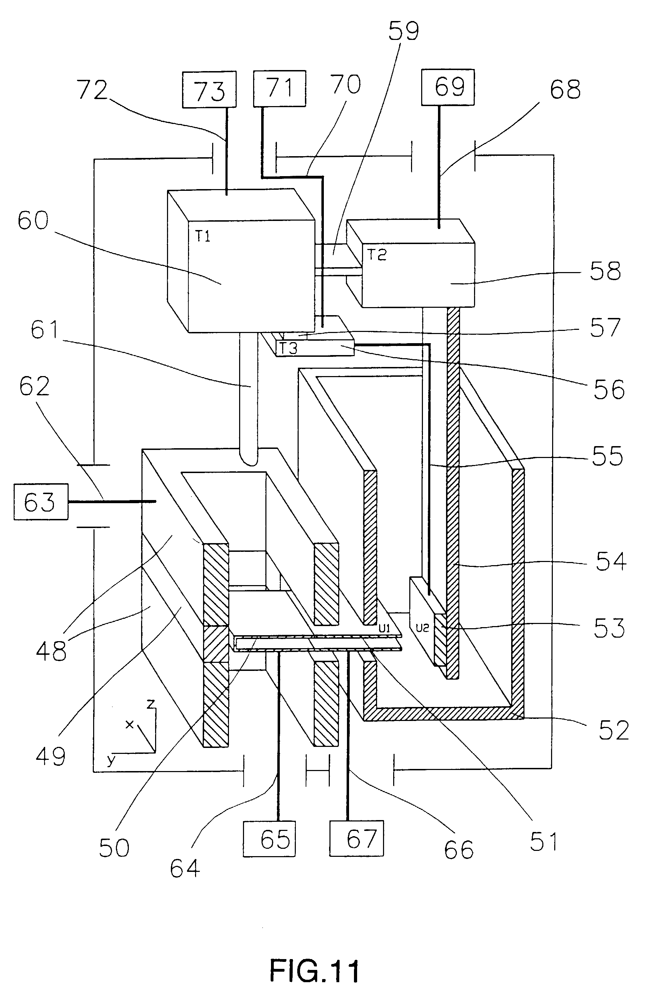

The preferred embodiment of the high throughput mass spectrometer with a cryogenic detector array is shown schematically in FIG. **11.** The two basic components are: a magnet consisting of two superconducting rectangular Helmholtz coils **48**, creating a homogeneous magnetic field B parallel to the z-axis, and a cryogenic detector array **53** inside a superconducting magnetic shield **52**. All components are in the same vacuum system and are cooled by a combined cryogenic system **60**. **T1** is the operating temperature of the superconducting magnet, **T2** the operating temperature of the cryogenic detectors which are cooled by an additional cryostat **58**, and **T3** the operating temperature of the preamplifiers.

The sample to be analyzed is placed in the pre-acceleration chamber **51** where the macromolecules are volatilized and ionized by an external mechanism **67** with feed through **66**, e.g. laser beam in the case of MALDI or a capillary in the case of ESI. The ionized macromolecules are pre-accelerated to a kinetic energy of typically a few 100 eV and enter the mass separator **50** which is placed inside of the magnet with a magnetic field of the order of a few Tesla, depending on the selected mass range. Both, pre-acceleration chamber 51 and mass separator are on an electrostatic potential of **U1** maintained by a high voltage supply **65** via current lead **64**, however, electrically insulated from the magnet. The superconducting magnet consists of two superconducting rectangular Helmholtz coils **48** separated by a spacer **49** of superconducting material for the magnetic field at the position of the mass separator **50** to be as parallel to the z-axis as possible. The magnet is cooled by the cryostat **60** via the thermal contact **61** to its operating temperature **T1**. Reference number **73** designates the supply of cryogenic liquids and **72** the transfer line **72**. The current of the superconducting magnet is supplied by a current source **63** through the leads **62**.

In the perpendicular magnetic field B of the magnet, the charged macromolecules move on circular paths with a radius of curvature inversely proportional to their masse/charge ratio (see FIG. 10). After describing exactly a half circle, the mass separated macromolecules enter a post-acceleration stage and finally reach the cryogenic detector array **53**. The kinetic energy of the macromolecules at the position of the cryogenic detector array is e·(U2-U1), where e is the unit charge, **U1** is the electric potential of the mass separator and **U2** the electric potential of the cryogenic detector array which will usually be at ground potential. In order to protect the cryogenic detector array from the strong magnetic stray fields, it is placed in magnetic shielding **52,** preferably also of a superconducting material. The cryogenic detector array **53** is connected thermally to the cold finger **54** which is cooled by the cryostat **58** to the operating temperature **T2** of the cryogenic detector array **53**. The cryostat **58** is connected to the major cryostat **60** for pre-cooling and liquification of $^3$He in the case of an embodiment of **58** as a $^3$He-cryostat. The cryostat is controlled by a temperature controll system and pumps **69** via the connection **68**. The cryogenic detector array is biased and read out electronically by an electronic pre-amplifier system **56** which can be cooled to its operating temperature **T3** via the thermal link **57** to the cryostat **60**. The output of the pre-amplifiers is connected to the data acquisition system **71** via connection **70**.

In the following, the response of this high-throughput embodiment of the invention to various design and operating parameters is illustrated by presenting the results of various calculations. Basically, a 2-dimensional computer code has been used [23] which allows the calculation of electric and magnetic fields and the determination of trajectories of macromolecules in those fields. The trajectory calculations yielded both spatial and temporal information of the macromolecule at any given time step. Unless noted otherwise, the parameters of this particular embodiment in the calculation were: geometry as shown in FIG. **10**, where the most relevant dimension is the inner dimensiion of the mass separator **50** (64 cm x 32 cm). With those dimensions, and the configuration as shown in FIG. **10**, the macromolecule masses between M=400000 amu and M=800000 amu are detected by the cryogenic detector array for a magnetic field B=6.5 Tesla, a pre-acceleration voltage $U_{pre}$=200 V and a post acceleration voltage $U_{post}$=50 kV. The spatial detector resolution is assumed to be $\delta x$=0.1 mm and the temporal detector resolution 100 nsec. Both values are typical values for cryogenic particle detectors and are technologically realizable. For calculations where the mass of the macromolecule was fixed, an intermediate value of M=600000 amu was chosen.

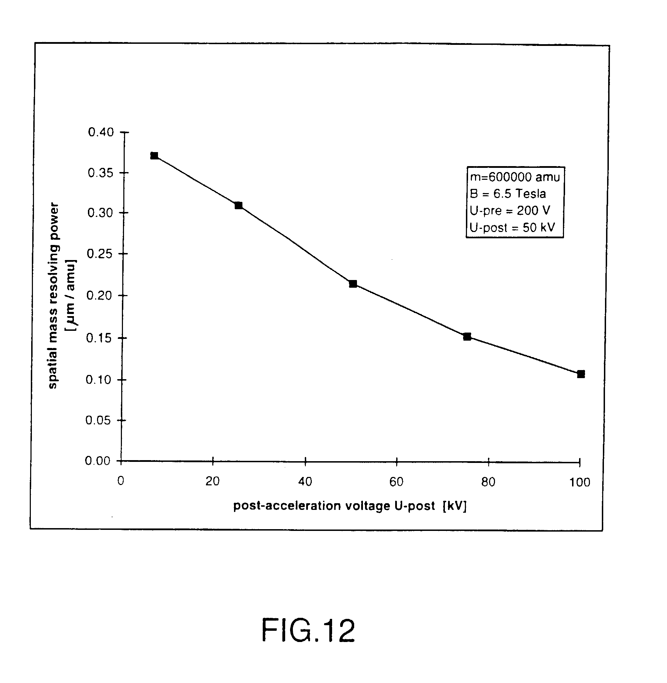

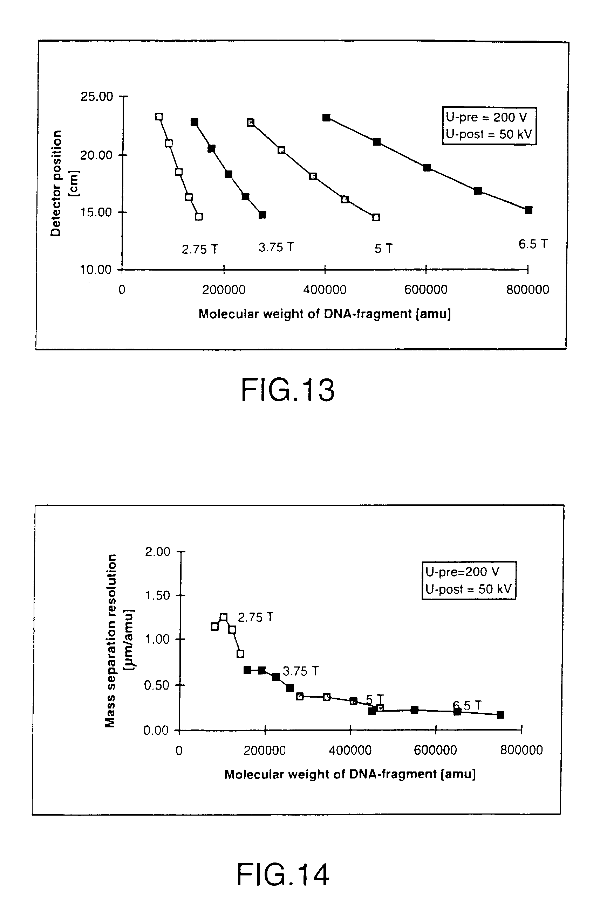

The mass separation of the macromolecules is determined by the combination of the magnetic field strength B and the pre-acceleration voltage $U_{pre}$. A post-acceleration voltage $U_{post}$ is required to accelerate the macromolecules to a sufficiently high kinetic energy of a few 10 keV, in order to be detectable by the cryogenic detector array. Increasing $U_{post}$ will make detection of the macromolecules by the cryogenic detector array easier, but, as is apparent from FIG. **12**, the macromolecules are then focussed to a smaller region of the cryogenic detector array, reducing the spatial mass resolving power accordingly. In FIG. **13** the various mass ranges for different values of the magnetic field strengths are shown. For a given magnetic field strength, the mass range of macromolecules reaching the cryogenic detector array is finite because of the finite exit window of the mass separator as shown in FIG. **10**. As is apparent from FIG. **13**, larger magnetic fields yield larger mass ranges which can be detected simultaneously by the cryogenic detector array. However, because those masses are spread out on a length of 10 cm, the spatial mass separation resolution decreases for increasing masses, as shown in FIG. **14**. Magnetic fields of 6.5 T can be readily achieved with superconducting coils, however the relatively large area will be technologically challenging. A good homogeneity of the magnetic field is required, small inhoinogeneities can be corrected for by considering the two important calibration curves of this preferred embodiment of the invention: the position calibration curve $M_{x-cal}(x)$ and the time calibration curve $M_{t-cal}(t)$. In order to obtain a good mass resolution, a short and long time stability of the magnetic field will be important.

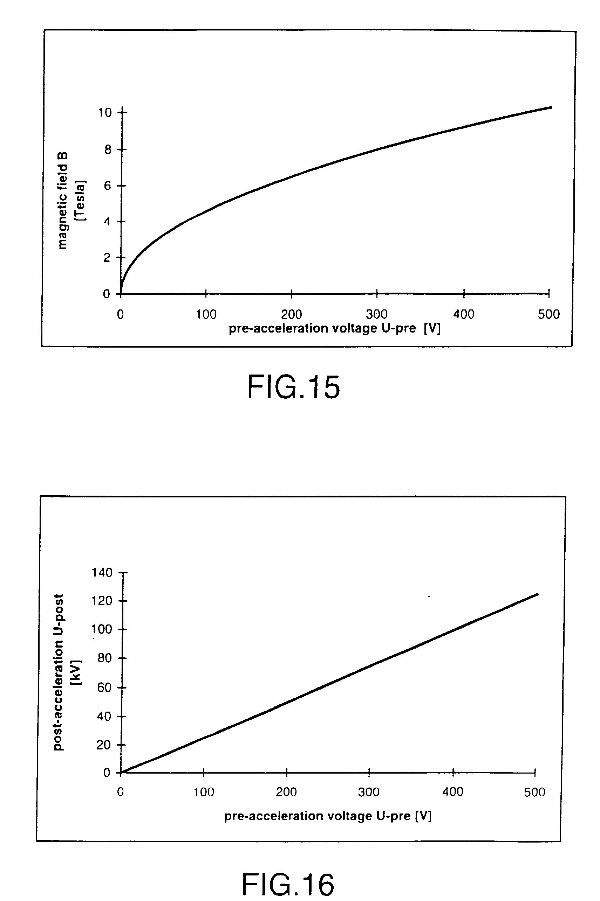

Another critical factor for achieving a good mass resolution with this high throughput mass spectrometer is the quality, of the ionized macromolecular beam entering the mass separator (reference number **65** in FIG. **10**). This beam will have to be highly collimated and should be monoenergetic. It is to be expected that this high beam quality would be achieved more easily for higher pre-acceleration voltages $U_{pre}$. The three operating parameters magnetic field B, $U_{pre}$ and $U_{post}$ cannot, however, be chosen independently for a given mass range. In FIG. **15** the required magnetic field strength B to detect the mass range between 400000 and 800000 amu at the position of the cryogenic detector array is given as a function of $U_{pre}$, and in FIG. **16** the corresponding post-acceleration value $U_{post}$ is given as a function of $U_{pre}$ for the same mass range. If, for instance, one selects a pre- acceleration voltage of 400 V, then the magnetic field would have to be set to a value of 8 Tesla (FIG. **15**) and $U_{post}$ to a value of 100 kV (FIG. 16), in order to detect the mass range between 400000 and 800000 amu at the position of the cryogenic detector array. An advantage of this preferred embodiment of the invention is that within a fixed geometry, a mass range of macromolecules can be scanned for masses ranging from virtually zero to a mass limited only by the highest permissable magnetic field. In addition, the pre- and post-acceleration voltages can be chosen such to optimize the overall performance.

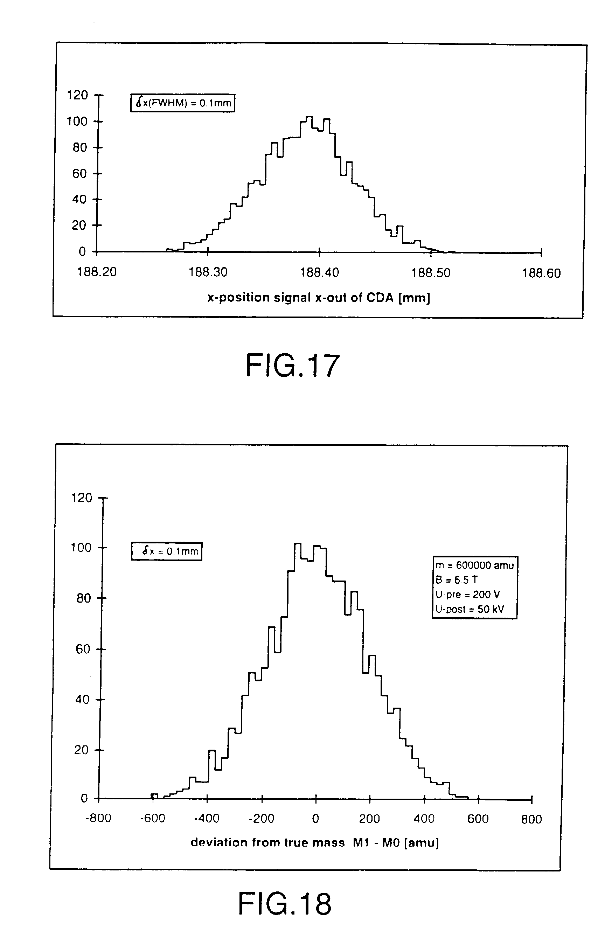

The mass of the macromolecules can be directly determined from the spatial mass resolving power of the cryogenic detector array (see FIG. **14**). In this particular numerical model, the spatial mass resolving power of the cryogenic detector array is 0.2 μm/amu (see FIG. **14**). If one wants to realize a mass resolution of, say, 100 amu (which would be required if one were to measure the mass of large DNA-fragments for DNA-sequencing, where the base mass is approximately 300 amu, see below) one would need a spatial resolution of the cryogenic detector array of 25 μm. This would require about 4000 individual detectors for a cryogenic detector array of 10 cm length in the embodiment shown in FIG. **7**. When using the embodiment of a cryogenic detector array as shown in FIG. **8** or FIG. **9**, one could obtain a spatial resolution $\delta x=0.1$ mm with phonon sensors spaced 1 mm apart. Then only 100 phonon sensors would be required to span the 10 cm. A Monte-Carlo calculation was performed to investigate the mass resolution properties of the various embodiments of this invention. The "true" values $x_{stop}$ of 2000 macromolecules with a mass of $M_0=600000$ amu were randomized by a gaussian distribution with a FWHM (full width at half maximum) of $\delta x=0,1$mm, simulating the cryogenic detector array output $x_{out}$. In FIG. **17** the spatial x-position signal distribution of the cryogenic detector array output $x_{out}$ is shown. With the calculated machine's calibration curve $M_{x-cal}(x)$, obtained by calculating the trajectories of the corresponding macromolecules, a mass $M_1$ was determined. In FIG. **18** the corresponding distribution of the mass error $M_1 - M_0$ is shown. The FWHM of this mass error distribution is about 500 amu and would be insufficient for DNA-sequencing. However, this mass resolution can be improved by using a time-of-flight strategy and the embodiment of a pulsed emission mode of this high-throughput mass spectrometer. This will be the scope of the following section.

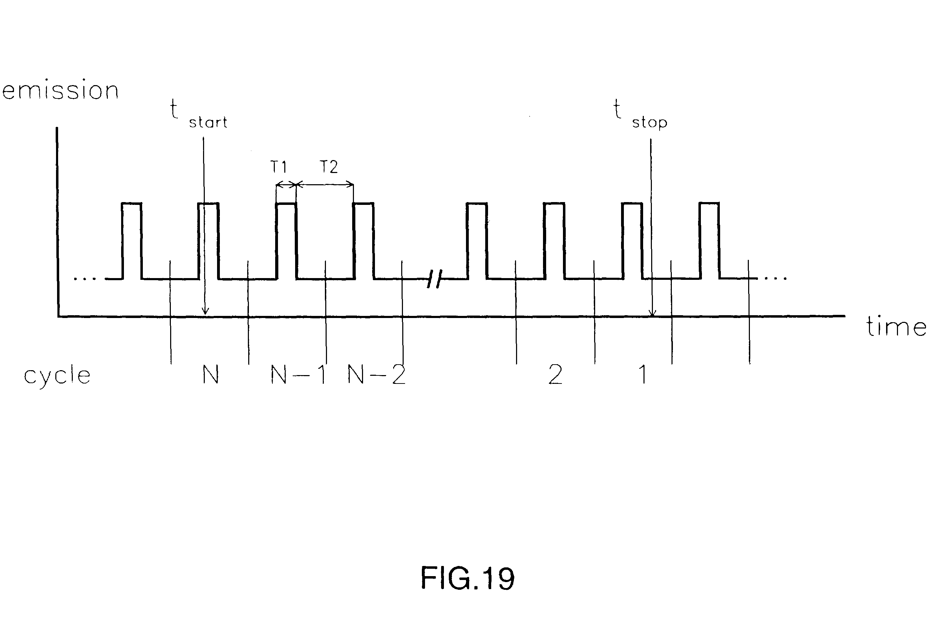

In a time-of-flight mass spectrometer, one measures the time difference between the time of emission $t_{start}$ of a particle and its time of impact $t_{stop}$ at a given detector. As the typical time-of-flight of a macromolecule in our numerical model is 5 msec, one could emit a pulse of macromolecules every $T_2=10$ msec with a pulse length of, say, $T_1=100$ nsec. This would yield a duty cycle $T_1/(T_1+T_2)$ of $10^{-5}$, and accordingly a low throughput of the device. The idea of this particular embodiment of operating mode is to perform many time-of-flight measurements in parallel by profiting from the spatial separation of the trajectories in the magnetic field. The pulsed emission is shown in FIG. **19**. The stop signal $\mathbf{t}_{stop}$ is measured by the cryogenic detector array, but the starting time $\mathbf{t}_{start}$ of the corresponding event remains to be determined. This is done by using the spatial separation of the trajectories as follows: As is shown in FIG.**19**, the macromolecules are emitted in pulses with a pulse length $\mathbf{T_1}$ and a non-emission pause $\mathbf{T_2}$. Such an emission pulse can be achieved by a corresponding laser pulses in the MALDI scheme, or with switching the electro-optics in the pre-acceleration phase. With the knowledge of the two calibration curves of the proposed embodiment - the position calibration curve $M_{x-cal}(x)$ and the time calibration curve $M_{t-cal}(t)$ - the mass of macromolecule can be reconstructed in the following way. The starting point are the two output signals of the cryogenic detector array:

$$x_{out} = x_{stop} + \delta x$$

$$t_{out} = t_{stop} + \delta t$$

where $x_{stop}$ is the true position of absorption and $t_{stop}$ the true time of impact at the cryogenic detector array, and $\delta x$ and $\delta t$ are deviations from the true values owning to the spatial and time resolution of the cryogenic detector array, respectively. From the value $x_{out}$ one determines a first guess $M_1$ of the mass by using the position calibration curve:

$$M_1 = M_{x\text{-}cal}(x_{out})$$

Entering this value $M_1$ into the inverse $M_{t\text{-}cal}^{-1}$ of the time calibration curve one obtains a first guess $t_1$ of the emission time:

$$t_1 = t_{out} - M_{t\text{-}cal}^{-1}(M_1)$$

Now, knowing that the macromolecule must have been emitted during one of the emission pulses, one can identify the corresponding cycle number N in which the macromolecule was emitted (see FIG. **19**).

$$N = 1 + \frac{t_1}{T_1 + T_2}$$

and obtain a better second guess $t_2$ of the emission time by identifying $t_{start}$ with the leading edge of the emission pulse:

$$t_2 = (N - 1)(T_1 + T_2) + \frac{T_2}{2}$$

The value of $t_2$ is, of course, only known to a precision corresponding to the pulse length $T_1$. Using again the time calibration curve, one finally arrives at the TOF value $M_2$ of the mass:

$$M_2 = M_{t\text{-}cal}(t_{out} - t_2)$$

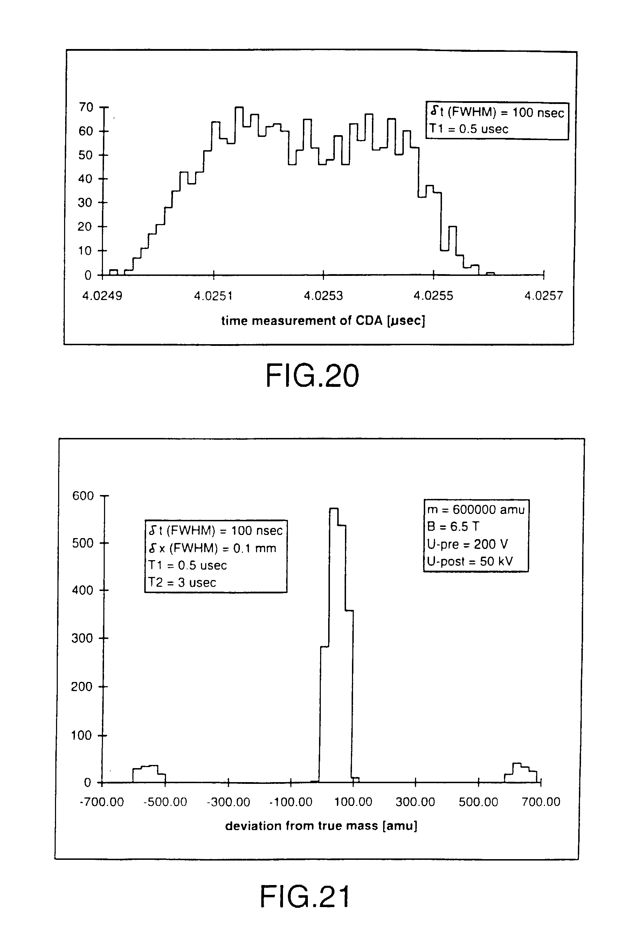

In this TOF operating mode, the precision of the value $M_2$ of the macromolecule is determined by the length $T_1$ of the emission pulse and the time resolution $\delta t$ of the crvogenic detector array. In this pulsed operating mode, the spatial resolution $\delta x$ of the cryogenic detector array is only required for reconstructing the cycle number N and does not enter the mass resolution directly. In Fig. **20** the Monte Carlo distribution of the detector time-of-arrival signal $\mathbf{t}_{stop}$ is shown for pulse width of $\mathbf{T}_1$ of 0.5 µsec and a time resolution $\delta t$ of the cryogenic detector array of 100 nsec.

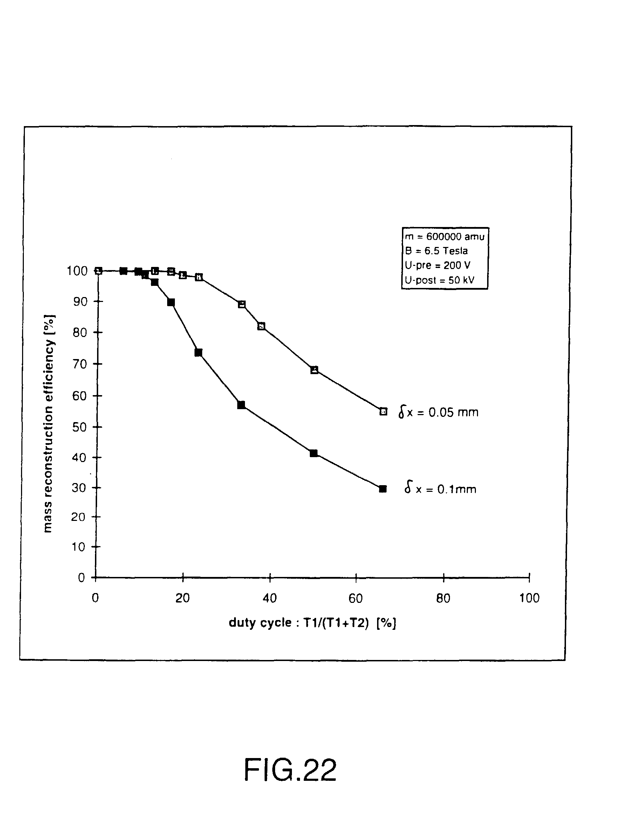

In FIG. **21**, the distribution of the TOF reconstructed mass is shown, which was obtained as follows: in the Monte Carlo calculation, each event was characterized by its corresponding value $x_{out}$ (FIG. **17**) and $t_{stop}$ (FIG. **20**). Using the mass reconstruction described above, the mass value $M_2$ was determined by entering those two variables into the corresponding calibration curves. The distribution of the mass determination error $M_2$-$M_0$ has a central peak with a FWHM of 100 amu corresponding to correctly identified cycle numbers N and two side peaks with the same FWHM corresponding to falsely identified neighbouring emission cycles owing to a "leakage" of values of $t_1$ into those emission cycles (see FIG. **19**). The reason for this "leakage" is the limited spatial resolution $\delta x$ of the cryogenic detector array and the correspondingly, small value $T_2$ of the pause in the pulse sequence. Increasing $T_2$ for a given value of $\delta x$ reduces the number of erraneously constructed events. However, the duty cycle will then be reduced accordingly. In FIG. **22**, the calculated mass reconstruction efficiency is shown as a function of duty cycle for two values of spatial resolution $\delta x$, where the mass reconstruction efficiency is defined to be the ratio of correctly to uncorrectly reconstructed events.

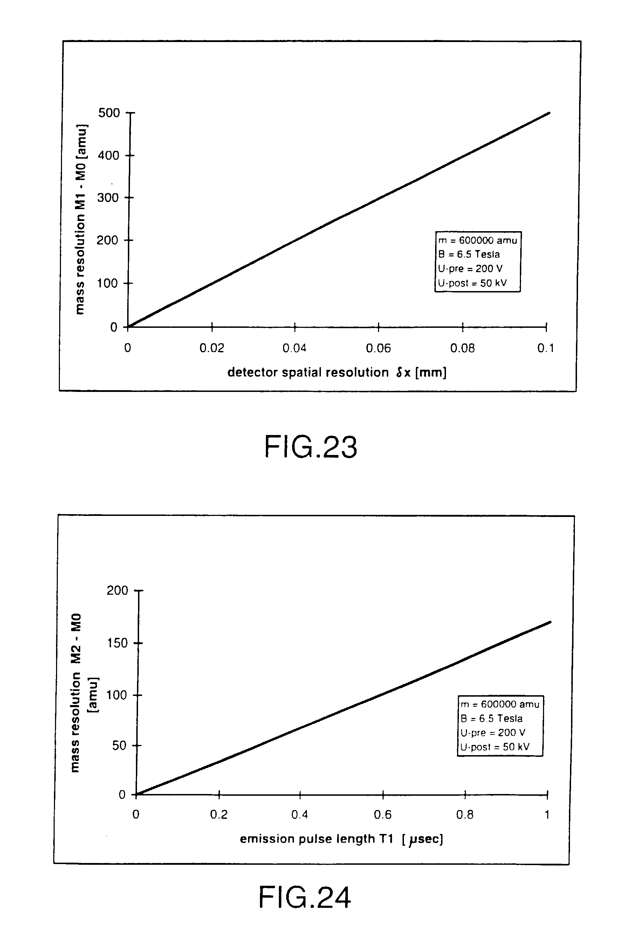

The mass resolution of the directly measured mass value $M_1$ can be improved by increasing the spatial resolution of the crvogenic detector array (i.e. by making the corresponding value of $\delta$ smaller), as is shown in FIG. **23**. The corresponding mass resolution of the TOF deduced mass distribution $M_2$ can be improved by reducing the emission pulse length $T_1$, as shown in FIG. **24**. However, in the latter case the mass resolution will be more likely be determined by the finite time resolution of the cryogenic detector array. The calculated mass resolutions above were obtained under the assumption that the ionized, pre-accelerated beam of macromolecules enters the mass seperator perfectly collimated, with all macromolecules having the same kinetic energy of 200 eV. This, of course, will not be true in a real device, and it will be one of the main technological challenges in constructing this preferred embodiment of the invention to fulfill those ideal initial beam conditions as much as possible.

A particular use of this preferred embodiment of the invention is for high throughput DNA-sequencing. The goal of DNA-sequencing is to determine the sequence of the four bases making up the DNA alphabet, A (adenine), G (guanine), T (thymine) and C (cytosine). In the molecular biology laboratory, aliquots containing DNA-sequence ladders are prepared according to either the Sanger or the Maxam-Gilbert. Taking the Sanger strategy as example, four aliquots of the same part of the DNA are produced which can be labeled by the four bases A, G, T and C, each aliquot containing information on the corresponding base position in the specific part of DNA in question. Conventionally, the macromolecules are sorted according to their length for each of the four aliquots by using the technique of gel-electrophoresis. There, the macromolecules migrate in a gel which is placed in an electric field. For a given duration, the shorter fragments migrate farther than the longer fragments, leading to a separation of the macromolecules accordingly. The major disadvantage of gel-electrophoresis is the long time (of the order of hours) required for the macromolecules to migrate through the gel. Typically a good equipped laboratory can sequence of the order of 10000 bases per day.

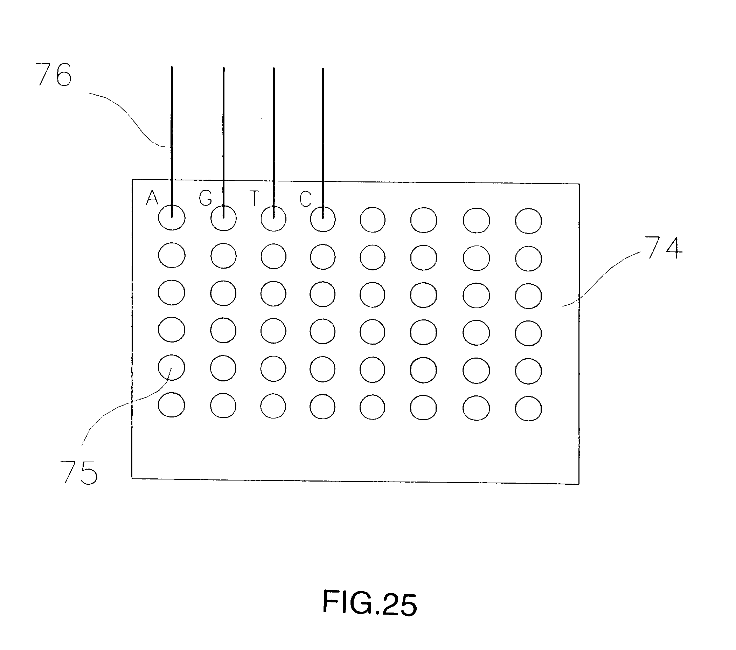

An intrinsically much faster method is to separate the DNA-fragments according to their masses by using mass spectrometry. As has been shown above, in the preferred embodiment of this invention, large DNA-fragments of mass 600000 amu can be analyzed with a mass resolution of the order of 100 amu. In the following, the rate of analyzing the aliquots of the equivalent of the human genome consisting of $3 \cdot 10^9$ bases is estimated for this preferred embodiment. Because of the single particle counting property of the cryogenic detector array, pile-up has to be prevented. One has, hence, to assure that only a small number of macromolecules will reach the detector array at any given time. Then the single particle counting property, of the cryogenic particle detector will allow a one-to-one identification of each set of detector values ($x_{stop}, t_{stop}$) to a unique emission cycle of the pulsed emission. Although heavier molecules from an earlier emission event will arrive the cryogenic detector array later than a lighter molecule from a later emission event, they will never reach the same position of the detector, because each trajectory, is unique for each mass/charge ratio. It is one advantage of this preferred embodiment that multiple charged macromolecules can be distinguished by their different response to the operating parameters. If one places the DNA-fragment aliquots to be analyzed on a chip as illustrated in FIG. 25, the emission of each aliquot can be synchronized to the emission pulse of the mass spectrometer. Each mass value measured by the cryogenic detector array can then be correlated to a specific aliquot on the chip. We again take the mass range of the spectrometer to be between 400000 amu and 800000 amu and assume the mass resolution to be sufficient to separate bases, i.e. $\Delta m < 300$ amu. Because of the finite resolution of the system, we assume that 100 events are required per macromolecule to unambiguously identify its mass. Within this given mass range, a sequencing ladder consisting of 1300 bases can be reconstructed for the four aliquots denoted by A, G, T and C in FIG. 25. In order to reconstruct successfully, the mass from the detector values ($x_{stop}, t_{stop}$), and to correlate the macromolecul to the aliquot on the chip it originated from, only one macromolecule should hit in average the detector at a given position and time for no pile-up to occur. A given aliquot would therefore have to be analyzed a 100 times. If the emission pulse period is chosen to be 100 μsec, then one can reconstruct the sequence of the 1300 bases of the given DNA-strand in 100 x 4 x 100 μsec = 40 msec. The next four aliquots would correspond to macromolecule populations shifted by 1300 bases. Again it would take 40 msec for those 1300 bases to be sequenced. In one second the machine would therefore be capable to reconstruct $3.25 \cdot 10^4$ bases, or, in other words, the equivalent number of bases of the entire human genome consisting of $3 \cdot 10^9$ bases could be sequenced in $9.2 \cdot 10^4$ seconds, i.e. in 25 hours. If one were to sequence the human genome with one single gel-electrophoresis apparatus capable of sequencing 10000 bases a day, it would take 800 years in comparison. However, the separation of the sequence ladder is only one of the steps required in DNA-sequencing. The biochemical production of the sequence ladder is elaborate and will require much more than a few hours for the entire human genome. Nevertheless, sample arrays can be prepared and stored as indicated in FIG. 25 by different laboratories in parallel and analyzed by the preferred embodiment of this invention in a very short time.

The entire amount of DNA required is also small: a sample array as shown in FIG. 25 would have to consist of $3 \cdot 10^9 / 1300 = 2.3 \cdot 10^6$ aliquots, which can be put in the form of an array of 1500 x 1500 aliquots. In our example, the four aliquots designated by A,G,T and C together contain the complete ladder of 1300 macromolecules with an average mass of 600000 amu. The total mass of macromolecules hitting the cryogenic detector array per emission cycle (i.e. per aliquot) is therefore (1300/4) x 600000 amu. Because one has to analyze the same aliquot a 100 times to get a good estimate for the mass this value has to be multiplied by 100. In addition, because the overall volatilizing and ionizing efficiency is probably not better than $10^{-4}$, the amount would have again to be multiplied by a factor of $10^4$. Per aliquot we therefore obtain (1 amu = $1.66 \cdot 10^{-24}$ g):

$$\text{amount of DNA per aliquot} = 10^4 \cdot 100 \cdot \frac{1300}{4} \cdot 600000 \text{ amu} = 0.32 \cdot 10^{-9} \text{ g}$$

Multiplying by the number of aliquots on the sample array ($2.3 \cdot 10^6$) one gets for the total amount of DNA required on the sample array:

$$\text{total amount of DNA on sample array} = 2.3 \cdot 10^6 \text{ x } 0.32 \cdot 10^{-9} \text{ g} = 0.74 \cdot 10^{-3} \text{ g}$$

Now let us estimate the size of the sample array: As each aliquot consists basically of a solvent with the DNA dissolved to a factor of $10^{-4}$, the mass of a aliquot would be $3.2 \cdot 10^{-6}$g. If, for simplicity, we assume the density to be the density of water, this mass would correspond to a volume of $3.2 \cdot 10^{-6} cm^3$, or, alternatively to a aliquot radius of 91 $\mu$m. The aliquots could therefore be spaced at a distance of 0.2 mm each. The overall size of the sample array would hence be:

$$\text{sample array size} = (1500 \cdot 0.2 \text{ mm}) \text{ x } (1500 \cdot 0.2 \text{ mm}) = 30 \text{ cm x } 30 \text{ cm}$$

The total amount of DNA accumulated on the cryogenic detector array during the sequencing of the entire humane genome as described above would be $7.4 \cdot 10^{-11}$ g, which corresponds to a film thickness of 7.4 nm for a cryogenic detector array of 10 cm length and a molecular beam height of 1 mm. A polymer film of this thickness will hardly alter the phonon sensitivity of the cryogenic detector.

In the calculations presented above, DNA-sequencing was as an application of the preferred embodiment of the invention. It goes without saying that the description above also apply for proteins, peptides, polymers or any other macromolecule.

While the forms of the invention herein constitute presently preferred embodiments, many others are possible. It is not intended herein to mention all of the possible equivalent forms or ramifications of the invention.

## REFERENCES

1. M. Barber et al., Anal. Chem., 54 (1982) 645 A.

2. J.E. Bruce et al., Rapid Commun. Mass Spectrom., 7 (1993) 914.

3. S.M. Michael et al., Anal. Chem., 65 (1993) 2614.

4. M. Karas and F. Hillenkamp, Anal. Chem. 60 (1988) 2299.

5. R.T. Melver et al., Int. J. Mass Spectrom. Ion Processes, 132 (1994) L1.

6. J.A. Castoro and C.L. Wilkins, Anal. Chem. 65 (1993) 2621.

7. K.J. Wu et al., Rapid Commun. Mass Spectrom., 7 (1993) 142.

8. P. Williams, Int. J. Mass Spectrom. Ion Processes, 131 (1994) 335.

9. D.F. Hunt et al., Proc. Natl. Acad. Sci. U.S.A. 83 (1986) 6233.

10. A.L. Cox et al., Science 264 (1994) 716.

11. J. Linhard and M. Scharf, Phys. Rev., 124 (1961) 128.

12. M.W. Senko and F.W. McLafferty, Annu. Rev. Biophys. Biomol. Struct. 23 (1994) 763.

13. Q.Xie, Science 256 (1992) 225.

14. "Advances in DNA Sequencing Technology", R.A. Keller (ed.), SPIE 1891, (1993).

15. D.C. Schwartz and C.R. Cantor, Cell 37 (1984) 67.

16. M. Burmeister and L. Ulanovsky (ed.) Methods in Molecular Biology, Vol 12, "Pulsed-Field Gel Electrophoresis" (1992).

17. D. Twerenbold, Europhys.Lett., 1 (1986) 209.

18. H. Kraus et al., Europhys.Lett., 1 (1986) 161.

19. Proceedings of the 5.International Workshop on Low Temperature Detectors, University of California at Berkeley, July 29 - August 3, 1993. Special issue: Journal of Low Temperature Physics 93 (1993).

20. D. Twerenbold, Phys. Rev B., 34 (1986) 7748.

21. Y.DeCoulon, D. Twerenbold and J.-L. Vuilleumier, Nucl. Instr. and Meth., A294 (1990) 259.

22. N.E. Booth, Appl. Phys. Lett., 50 (1987) 293.

23. The calculations of the potential field lines and trajectories are based on the POISSON group of codes developed by R. Holsinger and K. Halbach of the Los Alamos National Laboratory.

## Claims

1. A mass spectrometer comprising:

means (14) for volatizing and charging macromolecules out of a condensed solution;
an evacuated receptacle (13) in which said macromolecules are volatized and charged by said means;
electro-optical means (19, 22) placed in said receptacle for accelerating said charged macromolecules;
a high voltage power supply (23) and electrical connections to said electro-optical means;
means for separating said macromolecules according to their mass to charge ratio;
a detector system for detecting said macromolecules;
characterized in that the detector system comprises a phonon sensitive cryogenic particle detector (17) comprising one or more absorbers (1) and one or more phonon sensors (2) for detecting the time of impact and/ or the position of impact of said accelerated macromolecules on the cryogenic detector; whereby the absorbed kinetic energy of said impacting macromolecules is converted into phonons (6) excited in said absorber and whereby said phonon sensors convert said phonons into an electrical signal;
a preamplifier system (10) for converting said electronics signal of said cryogenic particle detector into a low impedance signal for further data processing; and
a cryostat with a cold finger (17) to which said cryogenic particle detector is thermally connected.

2. The mass spectrometer as claimed in claim 1 characterized in that said means for separating said macromolecules according to their mass to charge ratio is an evacuated mass separation receptacle (13) of sufficient length in which said macromolecules are separated according to their mass to charge ratio dependent velocites and the arrival time of said macromolecules is detected at the end of said evacuated mass separation receptacle with said phonon sensitive cryogenic particle detectors (42).

3. The mass spectrometer as claimed in claim 1 characterized in that said means for separating said macromolecules according to their mass to charge ratio is an evacuated mass separation receptacle of sufficient length placed in one or more quadrupole mass filters.

4. The mass spectrometer as claimed in claim 1 characterized in that said means for separating said macromolecules according to their mass to charge ratio is an evacuated mass separation receptacle surrounded by a means for generating a static magnetic field in which said macromolecules are separated according to their mass to charge ratio dependent trajectories in said static magnetic field.

5. The mass spectrometer as claimed in claim 4 characterized in that said means for generating a static magnetic field is a magnet (48) and the detector system consists of an array (53) of phonon sensitive cryogenic detectors, where said mass separation receptacle and a feed through (66) further comprised in said mass spectrometer are on an electrical potential different from the electrical potential of the cryogenic particle detector array and, in which the region between the feed through and said cryogenic particle detector array is shielded from the magnetic field of the magnet by a magnetic shield (52).

6. The mass spectrometer as claimed in any one of claims 1 to 5, characterized in that said absorber (42) and said phonon sensor (43) of said phonon sensitive cryogenic particle detectors are identical and in that said excited phonons (47) are directly converted into an electronic signal.

7. The mass spectrometer as claimed in any one of claims 1 to 5, characterized in that said absorber (42) of the cryogenic particle detector is single crystal silicon.

8. The mass spectrometer as claimed in any one of claims 1 to 5, characterized in that said absorber (42) is single crystal sapphire.

9. The mass spectrometer as claimed in any one of claims 1 to 5, characterized in that said absorber (42) is single crystal germanium.

10. The mass spectrometer as claimed in any one of claims 1 to 9, characterized in that said phonon sensors are superconducting tunnelling junctions (2) operated in the Giaever-mode; whereby said excited phonons (7) break Cooper pairs in the superconducting films (3,5) of said superconducting tunnelling junctions and produce excess quasiparticles; whereby said excess quasiparticles tunnel through the insulating barrier (4) of said superconducting tunnelling junctions and produce an excess quasiparticle current which constitutes said electronic signal; whereby a magnet field is applied parallel to the superconducting tunnelling junctions by a magnet in order to suppress the DC Josephson current.

11. The mass spectrometer as claimed in claim 10, characterized in that said superconducting tunnelling junctions (43) are deposited on top of large area superconducting films (44) with a superconducting energy gap larger than the corresponding superconducting energy gap cf said superconducting tunnelling junctions; whereby said excess phonons (47) first travel into said large area superconducting films where they break Cooper pairs and produce said excess quasiparticles which are trapped in said superconducting tunnelling junctions.

12. The mass spectrometer as claimed in any of claims 1 to 9, characterized in that said phonon sensors are superconducting transition edge thermometers; whereby said superconducting transition edge thermometers are operated at temperatures close to their phase transition temperature and biased electrically with a current slightly below their critical current; whereby said superconducting transition edge thermometers are heated due to said excess phonons; whereby said heating produces a temperature rise; whereby said temperature rise produces a superconducting to normal phase transition; whereby said phase transition produces a voltage signal which constitutes said electronic signal.

13. The mass spectrometer as claimed in any of claims 1 to 9, characterized in that said phonon sensors are superconducting kinetic inductance thermometers; whereby said superconducting kinetic inductance thermometers are operated at temperatures close to their phrase transition temperature and biased electrically with a current slightly below their critical current; whereby said superconducting kinetic inductance thermometers are heated up due to said excess phonons; whereby said heating produces a temperature rise; whereby said temperature rise produces a change in the London penetration depth; whereby said change in the London penetration depth produces a change in the inductance of the electronic circuitry; whereby said change inductance produces a voltage signal which constitutes said electronic signal.

14. The mass spectrometer as claimed in any of claims 1 to 9, characterized in that said phonon sensors are superconducting superheated granules; whereby said superconducting superheated granules are operated at temperatures close to their phase transition temperature; whereby the superconducting superheated granules are placed in an external magnetic field with a value slightly less than the critical magnetic field of said superconducting superheated granules; whereby said superconducting superheated granules are heated up due to said excess phonons; whereby said heating produces a temperature rise; whereby said temperature rise produces a superconducting to normal phase transition; whereby said phase transition produces a magnetic flux change owing to the penetration of magnetic field lines in the normal conducting granule; whereby said magnetic flux change produces a voltage signal in a pick up loop which constitutes said electronic signal.

15. The mass spectrometer as claimed in claim 14, characterized in that said superconducting superheated granules act as said absorbers and said phonon sensors.

16. The mass spectrometer as claimed in claims 14 or 15, characterized in that said superconducting superheated granules consist of small grains in a dielectric suspension.

17. The mass spectrometer as claimed in claims 14 or 15, characterized in that said superconducting superheated granules consist of two dimensional structures deposited onto substrate.

18. The mass spectrometer as claimed in claims 4 or 5, characterized in that said magnet is a superconducting magnet.

19. The mass spectrometer as claimed in any one of claims 10 to 17, characterized in that said superconducting structures are of niobium or an alloy of niobium.

20. The mass spectrometer as claimed in any one of claims 10 to 17, characterized in that said superconducting structures are of aluminium or an alloy of aluminium.

21. The mass spectrometer as claimed in any one of claims 10 to 17, characterized in that said superconducting structures are of tantalum or an alloy of tantalum.

22. The mass spectrometer as claimed in any one of claims 10 to 17, characterized in that said superconducting structures are of tin or an alloy of tin.

23. The mass spectrometer as claimed in any one of claims 10 to 17 characterized in that said superconducting structures are of indium or an alloy of indium.

**24.** The mass spectrometer as claimed in any one of claims 10 to 17, characterized in that said superconducting structures are of lead or an alloy of lead.

**25.** The mass spectrometer as claimed in claim 12, characterized in that said superconducting transition edge thermometer consist of indium/gold layers.

**26.** The mass spectrometer as claimed in any of claims 1 to 9, characterized in that said phonon sensors consist of semiconducting thermistors; whereby said semiconducting thermistors are biased by an electrical current; whereby said excess phonons heat the absorber; whereby said heating up leads to a temperature rise; whereby said temperature rise leads to a change in resistance of the semiconducting thermistor; whereby said temperature rise produces a voltage signal which constitutes said electronic signal.

**27.** The mass spectrometer as claimed in any one of claims 1 to 26, characterized in that said pre-amplifier (10) is integrated onto the substrate of said cryogenic particle detector.

**28.** The mass spectrometer as claimed in claim 27, as claimed in any one of said integrated pre-amplifier (10) consists of superconducting structures.

**29.** The mass spectrometer as claimed in any one of claims 1 to 28, characterized in that said means for volatilizing and charging macromolecules out of a condensed solution is based on the Matrix-Assisted Laser Desorption/Ionization (MALDI) technique.

**30.** The mass spectrometer as claimed in any one of claims 1 to 28, characterized in that said means for volatilizing and charging macromolecules out of a condensed solution is based on the Electron Spray Ionization (ESI) technique.

**31.** The mass spectrometer as claimed in any one of claims 1 to 28, characterized in that said means for volatilizing and charging macromolecules out of a condensed solution is based on the Fast Atom Bombardment (FAB) technique.

**32.** The mass spectrometer as claimed in any one of claims 1 to 28, characterized in that said means for volatilizing and charging macromolecules out of a condensed solution is based on the Plasma Desorption (PD) technique.

**33.** The mass spectrometer as claimed in any one of claims 1 to 28, characterized in that said means for volatilizing and charging macromolecules out of a condensed solution is based on the Surface-Enhanced Neat Desorption (SEND) technique.

**34.** The mass spectrometer as claimed in any one of claims 1 to 28, characterized in that said means for volatilizing and charging macromolecules out of a condensed solution is based on volatilizing said macromolecules by thermal heating of the substrate on which said macromolecules are deposited; whereby said volatile macromolecules are charged by separate means.

**35.** The mass spectrometer as claimed in claim 34, characterized by said means for volatilizing the macromolecules by thermal heating is produced by high frequency phonon emission techniques.

**36.** The mass spectrometer as claimed in claims 34 or 35, characterized in that said means for charging said volatile macromolecule is by photo ionization.

**37.** The mass spectrometer as claimed in claims 34 or 35, characterized in that said means for charging said volatile macromolecule is by X-ray ionization.

**38.** The mass spectrometer as claimed in any one of claims 5 to 37 characterized in that said mass spectrometer is operated in a pulsed operation mode; whereby said spatial position of impact yields a first guess of the mass of said macromolecule; whereby said first guess of the mass together with said time of impact yields the time of emission of said macromolecule; whereby said first guess of time of emission and said time of impact yields a second, more precises, determination of the mass of said macromolecules by calculating the time difference of said time of impact and said reconstructed time of emission.

**39.** The mass spectrometer as claimed in any one of claims 1 to 38, characterized in that the macromolecule aliquots (75) are on a two dimensional sample array (74).

**40.** The mass spectrometer as claimed in any one of claims 1 to 39, characterized by determining the mass of DNA-fragments for DNA-sequencing.

**41.** The mass spectrometer as claimed in any one of claims 1 to 39, characterized by determining the mass of proteins or protein-fragments for protein-sequencing.

**42.** The mass spectrometer as claimed in any one of claims 1 to 39, characterized by determining the mass of proteins or protein-fragments for protein-identification.

**43.** The mass spectrometer as claimed in any one of claims 1 to 39, characterized by determining the mass of polymers or polymers-fragments for polymer-identification.


**Patentansprüche**

**1.** Massenspektrometer, mit:

Mitteln (14) zum Verflüchtigen und Laden von Makromolekülen aus einer kondensierten Lösung;
einem evakuierten Behälter (13), in dem die Makromoleküle durch diese Mittel verflüchtigt und geladen werden;
elektrooptischen Mitteln (19, 22), die in dem Behälter angeordnet sind, um die geladenen Makromoleküle zu beschleunigen;
einer Hochspannungsenergiequelle (23) und elektrischen Verbindungen zu den elektrooptischen Mitteln;
Mitteln zum Separieren der Makromoleküle entsprechend ihrem Masse-Ladungs-Verhältnis;
einem Detektorsystem zum Detektieren der Makromoleküle;
**dadurch gekennzeichnet,** daß das Detektorsystem einen phononenempfindlichen, kryogenen Teilchendetektor (17), der einen oder mehrere Absorber (1) und einen oder mehrere Phononensensoren (2) zum Detektieren der Zeit des Aufpralls und/oder der Position des Aufpralls der beschleunigten Makromoleküle auf dem kryogenen Detektor enthält; wobei die absorbierte Energie der aufprallenden Makromoleküle in Phononen (6) umgewandelt wird, die den Absorber anregen, und wobei die Phononensensoren die Phononen in ein elektrisches Signal umwandeln;
ein Vorverstärkersystem (10) zum Umwandeln des elektrischen Signals von dem kryogenen Teilchendetektor in ein Signal mit niedriger Impedanz zur weiteren Datenverarbeitung; und
einen Kryostaten mit einem kalten Finger (17) aufweist, mit dem der kryogene Teilchendetektor thermisch gekoppelt ist.

**2.** Massenspektrometer nach Anspruch 1, **dadurch gekennzeichnet,** daß das Mittel zum Separieren der Makromoleküle entsprechend ihrem Masse-Ladungs-Verhältnis ein evakuierter Massenseparierungsbehälter (13) von ausreichender Länge ist, in dem die Makromoleküle entsprechend den von ihrem Masse-Ladungs-Verhältnis abhängigen Geschwindigkeiten separiert werden und die Ankunftszeit der Makromoleküle am Ende des evakuierten Massenseparierungsbehälters mit den phononenempfindlichen, kryogenen Teilchendetektor (42) detektiert wird.

**3.** Massenspektrometer nach Anspruch 1, **dadurch gekennzeichnet,** daß das Mittel zum Separieren der Makromoleküle entsprechend ihrem Masse-Ladungs-Verhältnis ein evakuierter Massenseparierungsbehälter von ausreichender Länge ist, der in einem oder mehreren Quadrupolmassenfilter angeordnet ist.

**4.** Massenspektrometer nach Anspruch 1, **dadurch gekennzeichnet,** daß das Mittel zum Separieren der Makromoleküle entsprechend ihrem Masse-Ladungs-Verhältnis ein evakuierter Massenseparierungsbehälter ist, der von einem Mittel zum Erzeugen eines statischen Magnetfeldes umgeben ist, in dem die Makromoleküle entsprechend den von ihrem Masse-Ladungs-Verhältnis abhängigen Bahnen in dem statischen Magnetfeld separiert werden.

**5.** Massenspektrometer nach Anspruch 4, **dadurch gekennzeichnet,** daß das Mittel zum Erzeugen eines statischen Magnetfeldes ein Magnet (48) ist und das Detektorsystem eine Matrix (53) aus phononenempfindlichen, kryogenen Teilchendetektoren aufweist, wobei sich der Massenseparierungsbehälter und eine außerdem in dem Massenspektrometer enthaltene Durchführung (66) auf einem elektrischen Potential befinden, das von dem elektrischen Potential der kryogenen Teilchendetektormatrix verschieden ist, und wobei das Gebiet zwischen der Durchführung und der kryogenen Teilchendetektormatrix durch eine magnetische Abschirmung (52) gegen das Magnetfeld von dem Magneten abgeschirmt ist.

6. Massenspektrometer nach einem der Ansprüche 1 bis 5, **dadurch gekennzeichnet,** daß der Absorber (42) und der Phononensensor (43) der phononenempfindlichen, kryogenen Teilchendetektoren identisch sind und daß die angeregten Phononen (47) direkt in ein elektrisches Signal umgewandelt werden.

7. Massenspektrometer nach einem der Ansprüche 1 bis 5, **dadurch gekennzeichnet,** daß der Absorber (42) des kryogenen Teilchendetektors Einkristallsilizium ist.

8. Massenspektrometer nach einem der Ansprüche 1 bis 5, **dadurch gekennzeichnet,** daß der Absorber (42) Einkristallsaphir ist.

9. Massenspektrometer nach einem der Ansprüche 1 bis 5, **dadurch gekennzeichnet,** daß der Absorber (42) Einkristallgermanium ist.

10. Massenspektrometer nach einem der Ansprüche 1 bis 9, **dadurch gekennzeichnet,** daß die Phononensensoren supraleitende Tunnelverbindungen (2) die im Giaever-Modus betätigt sind, wobei die angeregten Phononen (7) Cooper-Paare in den supraleitenden Schichten (3, 5) der supraleitenden Tunnelverbindungen brechen und überschüssige Quasiteilchen erzeugen, wobei die überschüssigen Quasiteilchen die isolierende Barriere (4) der supraleitenden Tunnelverbindungen durchtunneln und einen überschüssigen Quasiteilchenstrom erzeugen, der das elektrische Signal bildet; wobei ein Magnetfeld parallel zu den supraleitenden Tunnelverbindungen durch einen Magneten vorhanden ist, um den Josephson-Gleichstrom zu unterdrücken.

11. Massenspektrometer nach Anspruch 10, **dadurch gekennzeichnet,** daß sich die supraleitenden Tunnelverbindungen (43) auf der Oberseite von großflächigen, supraleitenden Schichten (44) befinden, mit einer supraleitenden Energielücke, die größer ist als die entsprechende supraleitende Energielücke der supraleitenden Tunnelverbindungen; wobei die überschüssigen Phononen (47) zuerst in die großflächigen supraleitenden Schichten wandern, wo sie Cooper-Paare brechen und die überschüssigen Quasiteilchen erzeugen, die in den supraleitenden Tunnelverbindungen eingefangen sind.

12. Massenspektrometer nach einem der Ansprüche 1 bis 9, **dadurch gekennzeichnet,** daß die Phononensensoren supraleitende Übergangsrandthermometer sind; wobei die supraleitenden Übergangsrandthermometer bei Temperaturen betrieben werden, die nahe bei deren Phasenübergangstemperatur liegen, und elektrisch mit einem Strom vorgespannt sind, der etwas unter deren kritischem Strom liegt; wobei die supraleitenden Übergangsrandthermometer durch die überschüssigen Phononen erhitzt werden; wobei die Erhitzung einen Temperaturanstieg erzeugt; wobei der Temperaturanstieg einen supraleitenden bis normalen Phasenübergang erzeugt; wobei der Phasenübergang ein Spannungssignal erzeugt, das das elektrische Signal bildet.

13. Massenspektrometer nach einem der Ansprüche 1 bis 9, **dadurch gekennzeichnet,** daß die Phononsensoren supraleitende kinetische Induktanzthermometer sind; wobei die supraleitenden kinetischen Induktanzthermometer bei Temperaturen betrieben werden, die nahe bei deren Phasenübergangstemperatur liegen, und elektrisch mit einem Strom vorgespannt sind, der etwas unter deren kritischem Strom liegt; wobei die supraleitenden kinetischen Induktanzthermometer durch die überschüssigen Phononen erhitzt werden; wobei die Erhitzung einen Temperaturanstieg erzeugt; wobei der Temperaturanstieg eine Änderung in der London-Penetrationstiefe erzeugt; wobei die Ladung in der London-Penetrationstiefe eine Änderung in der Induktanz der elektronischen Schaltung erzeugt; wobei die Änderung in der Induktanz ein Spannungssignal erzeugt, das das elektrische Signal bildet.

14. Massenspektrometer nach einem der Ansprüche 1 bis 9, **dadurch gekennzeichnet,** daß die Phononensensoren supraleitende, überhitzte Körner sind; wobei die supraleitenden, überhitzten Körner bei Temperaturen betrieben werden, die nahe bei deren Phasenübergangstemperatur liegt, wobei die supraleitenden, überhitzten Körner in einem externen Magnetfeld angeordnet sind, das einen Wert hat, der etwas kleiner ist als das kritische Magnetfeld der supraleitenden, überhitzten Körner; wobei die supraleitenden, überhitzten Körner durch die überschüssigen Phononen erhitzt werden; wobei die Erhitzung einen Temperaturanstieg erzeugt; wobei der Temperaturanstieg einen supraleitenden bis normalen Phasenübergang erzeugt; wobei der Phasenübergang durch die Penetration der Magnetfeldlinien in das normal leitende Korn eine Magnetflußänderung erzeugt; wobei die Magnetflußänderung in einer Aufnahmeschleife ein Spannungssignal erzeugt, das das elektrische Signal bildet.

15. Massenspektrometer nach Anspruch 14, **dadurch gekennzeichnet,** daß die supraleitenden, überhitzten Körner wie die Absorber und die Phononensensoren wirken.

16. Massenspektrometer nach Anspruch 14 oder 15, **dadurch gekennzeichnet,** daß die supraleitenden, überhitzten Körner kleine Körnchen in einer dielektrischen Suspension umfassen.

17. Massenspektrometer nach Anspruch 14 oder 15, **dadurch gekennzeichnet,** daß die supraleitenden, überhitzten Körner zweidimensionale Strukturen umfassen, die auf dem Substrat abgelagert sind.

18. Massenspektrometer nach Anspruch 4 oder 5, **dadurch gekennzeichnet,** daß der Magnet ein supraleitender Magnet ist.

19. Massenspektrometer nach einem der Ansprüche 10 bis 17, **dadurch gekennzeichnet,** daß die supraleitenden Strukturen Niob oder eine Nioblegierung enthalten.

20. Massenspektrometer nach einem der Ansprüche 10 bis 17, **dadurch gekennzeichnet,** daß die supraleitenden Strukturen Aluminium oder eine Aluminiumlegierung enthalten.

21. Massenspektrometer nach einem der Ansprüche 10 bis 17, **dadurch gekennzeichnet,** daß die supraleitenden Strukturen Tantal oder eine Tantallegierung enthalten.

22. Massenspektrometer nach einem der Ansprüche 10 bis 17, **dadurch gekennzeichnet,** daß die supraleitenden Strukturen Zinn oder eine Zinnlegierung enthalten.

23. Massenspektrometer nach einem der Ansrüche 10 bis 17, **dadurch gekennzeichnet,** daß die supraleitenden Strukturen Indium oder eine Indiumlegierung enthalten.

24. Massenspektrometer nach einem der Ansprüche 10 bis 17, **dadurch gekennzeichnet,** daß die supraleitenden Strukturen Blei oder eine Bleilegierung enthalten.

25. Massenspektrometer nach Anspruch 12, **dadurch gekennzeichnet,** daß das supraleitende Übergangsrandthermometer Indium/Goldschichten aufweist.

26. Massenspektrometer nach einem der Ansprüche 1 bis 9, **dadurch gekennzeichnet,** daß die Phononensensoren halbleitende Thermistoren enthalten; wobei die halbleitenden Thermistoren durch einen elektrischen Strom vorgespannt sind; wobei die überschüssigen Phononen der Absorber erhitzen; wobei die Erhitzung zu einem Temperaturanstieg führt; wobei der Temperaturanstieg zu einer Änderung des Widerstandes des halbleitenden Thermistors führt; wobei der Temperaturanstieg ein Spannungssignal erzeugt, das das elektrische Signal bildet.

27. Massenspektrometer nach einem der Ansprüche 1 bis 26, **dadurch gekennzeichnet,** daß der Vorverstärker (10) auf dem Substrat des kryogenen Teilchendetektors integriert ist.

28. Massenspektrometer nach Anspruch 27, **dadurch gekennzeichnet,** daß einer der integrierten Vorverstärker (10) supraleitende Strukturen aufweist.

29. Massenspektrometer nach einem der Ansprüche 1 bis 28, **dadurch gekennzeichnet,** daß das Mittel zum Verflüchtigen und Laden von Makromolekülen aus einer kondensierten Lösung auf der Matrix-Assisted Laser-Desorption/Ionization (MALDI) Technik basiert.

30. Massenspektrometer nach einem der Ansprüche 1 bis 28, **dadurch gekennzeichnet,** daß das Mittel zum Verflüchtigen und Laden von Makromolekülen aus einer kondensierten Lösung auf der Electron Spray Ionization (ESI) Technik basiert.

31. Massenspektrometer nach einem der Ansprüche 1 bis 28, **dadurch gekennzeichnet,** daß das Mittel zum Verflüchtigen und Laden von Makromolekülen aus einer kondensierten Lösung auf der Fast Atom Bombardment (FAB) Technik basiert.

32. Massenspektrometer nach einem der Ansprüche 1 bis 28, **dadurch gekennzeichnet,** daß das Mittel zum Verflüchtigen und Laden von Makromolekülen aus einer kondensierten Lösung auf der Plasma Desorption (PD) Technik basiert.

33. Massenspektrometer nach einem der Ansprüche 1 bis 28, **dadurch gekennzeichnet,** daß das Mittel zum Verflüchtigen und Laden von Makromolekülen aus einer kondensierten Lösung auf der Surface-Enhanced Neat Desorption (SEND) Technik basiert.

34. Massenspektrometer nach einem der Ansprüche 1 bis 28, **dadurch gekennzeichnet,** daß das Mittel zum Verflüchtigen und Laden von Makromolekülen aus einer kondensierten Lösung auf der Verflüchtigung der Makromoleküle durch thermische Erhitzung des Substrats basiert, auf dem die Makromoleküle abgelagert sind; wobei die flüchtigen Makromoleküle durch separate Mittel geladen sind.

35. Massenspektrometer nach Anspruch 34, **dadurch gekennzeichnet,** daß das Mittel zum Verflüchtigen der Makromoleküle durch thermische Erhitzung durch Hochfrequenz-Phononenemissionstechniken bewirkt ist.

36. Massenspektrometer nach Anspruch 34 oder 35, **dadurch gekennzeichnet,** daß das Mittel zum Laden des flüchtigen Makromoleküls durch Photoionisation bewirkt ist.

37. Massenspektrometer nach Anspruch 34 oder 35, **dadurch gekennzeichnet,** daß das Mittel zum Laden des flüchtigen Makromoleküls durch Röntgenionisation bewirkt ist.

38. Massenspektrometer nach einem der Ansprüche 5 bis 37, **dadurch gekennzeichnet,** daß das Massenspektrometer in einer gepulsten Betriebsart betrieben wird; wobei die räumliche Position des Aufpralls eine erste Schätzung der Masse des Makromoleküls ergibt; wobei die erste Schätzung der Masse zusammen mit der Zeit des Aufpralls die Zeit der Emission des Makromoleküls ergibt; wobei die erste Schätzung der Zeit der Emission und der Zeit des Aufpralls eine zweite, genauere Bestimmung der Masse der Makromoleküle durch Berechnung der Zeitdifferenz zwischen der Zeit des Aufpralls und der rekonstruierten Zeit der Emission ergibt.

39. Massenspektrometer nach einem der Ansprüche 1 bis 38, **dadurch gekennzeichnet,** daß die Makromolekül-Aliquoten (75) auf einer zweidimensionalen Probenanordnung (74) sind.

40. Massenspektrometer nach einem der Ansprüche 1 bis 39, **gekennzeichnet durch** die Bestimmung der Masse der DNA-Fragmente zur DNA-Sequenzierung.

41. Massenspektrometer nach einem der Ansprüche 1 bis 39, **gekennzeichnet durch** die Bestimmung der Masse von Proteinen oder Protein-Fragmenten zur Protein-Sequenzierung.

42. Massenspektrometer nach einem der Ansprüche 1 bis 39, **gekennzeichnet durch** die Bestimmung der Masse von Proteinen oder Protein-Fragmenten zur Protein-Identifizierung.

43. Massenspektrometer nach einem der Ansprüche 1 bis 39, **gekennzeichnet durch** die Bestimmung der Masse von Polymeren oder Polymer-Fragmenten zur Polymer-Identifizierung.

**Revendications**

1. Spectromètre de masse, comprenant :

   un dispositif (14) de volatilisation et de charge de macromolécules d'une solution condensée,
   un réceptacle évacué (13) dans lequel les macromolécules sont volatilisées et chargées par ledit dispositif,
   un dispositif électro-optique (19, 22) placé dans le réceptacle et destiné à accélérer les macromolécules chargées,
   une alimentation à haute tension (23) et des connexions électriques effectuées avec le dispositif électro-optique,
   un dispositif de séparation des macromolécules en fonction de leur rapport masse-charge, et
   un système détecteur des macromolécules,
   caractérisé en ce que le système détecteur comprend un détecteur cryogénique (17) de particules sensible à des phonons comprenant un ou plusieurs absorbeurs (1) et un ou plusieurs capteurs (2) de phonons destinés à détecter le temps d'impact et/ou la position d'impact des macromolécules accélérées sur le détecteur cryogénique, si bien que l'énergie cinétique absorbée des molécules présentant l'impact est transformée en phonons (6) excités dans l'absorbeur, et les capteurs de phonons transforment les phonons en un signal électrique, un système préamplificateur (10) destiné à transformer le signal électronique du détecteur cryogénique de particules en un signal de faible impédance permettant un traitement supplémentaire de données, et un cryostat ayant un doigt froid (17) auquel est raccordé thermiquement le détecteur cryogénique de particules.

2. Spectromètre de masse selon la revendication 1, caractérisé en ce que le dispositif de séparation des macromolécules en fonction de leur rapport masse-charge est un réceptacle évacué (13) de séparation de masse de longueur suffisante, dans lequel les macromolécules sont séparées d'après leur rapport masse-charge en fonction des vitesses, et le temps d'arrivée des macromolécules est détecté à l'extrémité du réceptacle évacué de séparation de masse avec les détecteurs cryogéniques de particules (42) sensibles aux phonons.

3. Spectromètre de masse selon la revendication 1, caractérisé en ce que le dispositif de séparation des macromolécules suivant leur rapport masse-charge est un réceptacle évacué de séparation de masse ayant une longueur suffisante, placé dans un ou plusieurs filtres quadripolaires de masse.

4. Spectromètre de masse selon la revendication 1, caractérisé en ce que le dispositif de séparation des macromolécules en fonction de leur rapport masse-charge est un réceptacle évacué de séparation de masse entouré par un dispositif générateur d'un champ magnétique statique dans lequel les macromolécules sont séparées d'après leur rapport masse-charge et leurs trajectoires dans le champ magnétique statique.

5. Spectromètre de masse selon la revendication 4, caractérisé en ce que le dispositif générateur d'un champ magnétique statique est un aimant (48) et le système détecteur comprend une matrice (53) de détecteurs cryogéniques sensibles aux phonons, dans lequel le réceptacle de séparation de masse et une traversée (66) incorporée en outre dans le spectromètre de masse sont à un potentiel électrique différent du potentiel électrique de la matrice de détecteurs cryogéniques de particules, et dans lequel la région comprise entre la traversée et la matrice de détecteurs cryogéniques de particules est protégée contre les champs magnétiques de l'aimant par un blindage magnétique (52).

6. Spectromètre de masse selon l'une quelconque des revendications 1 à 5, caractérisé en ce que l'absorbeur (42) et le capteur de phonons (43) des détecteurs cryogéniques de particules sensibles aux phonons sont identiques, et en ce que les phonons excités (47) sont directement transformés en un signal électronique.

7. Spectromètre de masse selon l'une quelconque des revendications 1 à 5, caractérisé en ce que l'absorbeur (42) du détecteur cryogénique de particules est du silicium monocristallin.

8. Spectromètre de masse selon l'une quelconque des revendications 1 à 5, caractérisé en ce que l'absorbeur (42) est un saphir monocristallin.

9. Spectromètre de masse selon l'une quelconque des revendications 1 à 5, caractérisé en ce que l'absorbeur (42) est du germanium monocristallin.

10. Spectromètre de masse selon l'une quelconque des revendications 1 à 9, caractérisé en ce que les capteurs de phonons sont des jonctions supraconductrices à effet tunnel (2) commandées en mode de Giaever, si bien que les phonons excités (7) brisent des paires de Cooper dans les films supraconducteurs (3, 5) des jonctions supraconductrices à effet tunnel et produisent des quasi-particules en excès, si bien que les quasi-particules en excès passent par effet tunnel dans la barrière isolante (4) des jonctions supraconductrices à effet tunnel et donnent un courant de quasi-particules en excès qui constitue le signal électronique, et dans lequel un champ magnétique est appliqué parallèlement aux jonctions supraconductrices à effet tunnel par un aimant afin que le courant continu Josephson soit réduit.

11. Spectromètre de masse selon la revendication 10, caractérisé en ce que les jonctions supraconductrices (43) à effet tunnel sont déposées à la partie supérieure de films supraconducteurs (44) de grande étendue ayant une bande d'énergie supraconductrice supérieure à la bande d'énergie supraconductrice correspondante des jonctions supraconductrices à effet tunnel, si bien que les phonons en excès (47) se déplacent d'abord dans les films supraconducteurs de grande étendue dans lesquels ils brisent des paires de Cooper et produisent les quasi-particules en excès qui sont piégées dans les jonctions supraconductrices à effet tunnel.

**12.** Spectromètre de masse selon l'une quelconque des revendications 1 à 9, caractérisé en ce que les capteurs de phonons sont des thermomètres supraconducteurs à flanc de transition, si bien que les thermomètres supraconducteurs à flanc de transition travaillent à des températures proches de leur température de transition de phase et sont polarisés électriquement par un courant légèrement inférieur à leur courant critique, dans lequel les thermomètres supraconducteurs de flanc de transition sont chauffés par les phonons en excès, dans lequel le chauffage produit une élévation de température, dans lequel l'élévation de température produit une transition d'une phase supraconductrice à une phase normale, et dans lequel la transition de phase produit un signal de tension qui constitue le signal électronique.

**13.** Spectromètre de masse selon l'une quelconque des revendications 1 à 9, caractérisé en ce que les capteurs de phonons sont des thermomètres supraconducteurs à inductance cinétique, dans lequel les thermomètres supraconducteurs à inductance cinétique travaillent à des températures proches de leur température de transition de phase et sont polarisés électriquement par un courant légèrement inférieur à leur courant critique, dans lequel les thermomètres supraconducteurs à inductance cinétique sont échauffés par les phonons en excès, dans lequel le chauffage produit une élévation de température, dans lequel l'élévation de température produit un changement de la profondeur de pénétration de London, dans lequel le changement de profondeur de pénétration de London produit un changement d'inductance du circuit électronique, et dans lequel le changement d'inductance provoque un signal de tension qui constitue le signal électronique.

**14.** Spectromètre de masse selon l'une quelconque des revendications 1 à 9, caractérisé en ce que les capteurs de phonons sont des granulés supraconducteurs surchauffés, dans lequel les granulés supraconducteurs surchauffés travaillent à des températures proches de leur température de transition de phase, dans lequel les granulés supraconducteurs surchauffés sont placés dans un champ magnétique extérieur de valeur légèrement inférieur à celle du champ magnétique critique des granulés supraconducteurs surchauffés, dans lequel les granulés supraconducteurs surchauffés sont chauffés par les phonons en excès, dans lequel le chauffage produit une élévation de température, dans lequel l'élévation de température produit une transition d'une phase supraconductrice à une phase normale, dans lequel la transition de phase produit un changement du flux magnétique du fait de la pénétration des lignes de champ magnétique dans le granulé conducteur normal, et dans lequel le changement de flux magnétique produit un signal de tension dans une boucle détectrice qui constitue le signal électronique.

**15.** Spectromètre de masse selon la revendication 14, caractérisé en ce que les granulés supraconducteurs surchauffés jouent le rôle d'absorbeurs et de capteurs de phonons.

**16.** Spectromètre de masse selon la revendication 14 ou 15, caractérisé en ce que les granulés supraconducteurs surchauffés sont constitués de petits grains dans une suspension diélectrique.

**17.** Spectromètre de masse selon la revendication 14 ou 15, caractérisé en ce que les granulés supraconducteurs surchauffés sont constitués de structures bidimensionnelles déposées sur un substrat.

**18.** Spectromètre de masse selon la revendication 4 ou 5, caractérisé en ce que l'aimant est un aimant supraconducteur.

**19.** Spectromètre de masse selon l'une quelconque des revendications 10 à 17, caractérisé en ce que les structures supraconductrices sont formées de niobium ou d'un alliage de niobium.

**20.** Spectromètre de masse selon l'une quelconque des revendications 10 à 17, caractérisé en ce que les structures supraconductrices sont formées d'aluminium ou d'un alliage d'aluminium.

**21.** Spectromètre de masse selon l'une quelconque des revendications 10 à 17, caractérisé en ce que les structures supraconductrices sont formées de tantale ou d'un alliage de tantale.

**22.** Spectromètre de masse selon l'une quelconque des revendications 10 à 17, caractérisé en ce que les structures supraconductrices sont formées d'étain ou d'un alliage d'étain.

**23.** Spectromètre de masse selon l'une quelconque des revendications 10 à 17, caractérisé en ce que les structures supraconductrices sont formées d'indium ou d'un alliage d'indium.

**24.** Spectromètre de masse selon l'une quelconque des revendications 10 à 17, caractérisé en ce que les structures supraconductrices sont formées de plomb ou d'un alliage de plomb.

25. Spectromètre de masse selon la revendication 12, caractérisé en ce que le thermomètre supraconducteur à flanc de transition est constitué de couches d'indium-or.

26. Spectromètre de masse selon l'une quelconque des revendications 1 à 9, caractérisé en ce que les capteurs de phonons sont constitués de thermistances semi-conductrices, dans lequel les thermistances semi-conductrices sont polarisées par un courant électrique, dans lequel les phonons en excès chauffent l'absorbeur, dans lequel le chauffage provoque une élévation de température, dans lequel l'élévation de température provoque un changement de résistance de la thermistance semi-conductrice, et dans lequel l'élévation de température provoque un signal de tension qui constitue le signal électronique.

27. Spectromètre de masse selon l'une quelconque des revendications 1 à 26, caractérisé en ce que le préamplificateur (10) est intégré sur le substrat du détecteur cryogénique de particules.

28. Spectromètre de masse selon la revendication 27, dans lequel le préamplificateur intégré (10) est constitué de structures supraconductrices.

29. Spectromètre de masse selon l'une quelconque des revendications 1 à 28, caractérisé en ce que le dispositif de volatilisation et de charge des macromolécules de la solution condensée met en oeuvre une technique de désorption-ionisation par laser assistée par le milieu (MALDI).

30. Spectromètre de masse selon l'une quelconque des revendications 1 à 28, caractérisé en ce que le dispositif de volatilisation et de charge des macromolécules de la solution condensée met en oeuvre une technique d'ionisation par pulvérisation électronique (ESI).

31. Spectromètre de masse selon l'une quelconque des revendications 1 à 28, caractérisé en ce que le dispositif de volatilisation et de charge de macromolécules de la solution condensée met en oeuvre une technique de bombardement rapide d'atomes (FAB).

32. Spectromètre de masse selon l'une quelconque des revendications 1 à 28, caractérisé en ce que le dispositif de volatilisation et de charge de macromolécules de la solution condensée met en oeuvre une technique de désorption dans un plasma (PD).

33. Spectromètre de masse selon l'une quelconque des revendications 1 à 28, caractérisé en ce que le dispositif de volatilisation et de charge de macromolécules de la solution condensée met en oeuvre une technique de désorption thermique renforcée en surface (SEND).

34. Spectromètre de masse selon l'une quelconque des revendications 1 à 28, caractérisé en ce que le dispositif de volatilisation et de charge de macromolécules de la solution condensée met en oeuvre la volatilisation des macromolécules par chauffage thermique du substrat sur lequel les macromolécules sont déposées, et dans lequel les macromolécules volatiles sont chargées par un dispositif séparé.

35. Spectromètre de masse selon la revendication 34, caractérisé en ce que le dispositif de volatilisation des macromolécules par chauffage est formé par des techniques d'émission de phonons à hautes fréquences.

36. Spectromètre de masse selon la revendication 34 ou 35, caractérisé en ce que le dispositif de charge de la macromolécule volatile est la photoionisation.

37. Spectromètre de masse selon la revendication 34 ou 35, caractérisé en ce que le dispositif de charge de la macromolécule volatile est l'ionisation par des rayons X.

38. Spectromètre de masse selon l'une quelconque des revendications 5 à 37, caractérisé en ce que le spectromètre de masse travaille en mode pulsé, la position spatiale d'impact permet une première estimation de la masse de la macromolécule, la première estimation de la masse et le temps d'impact donnent le temps d'émission de la macromolécule, la première estimation du temps d'émission et du temps d'impact donne une seconde détermination plus précise de la masse des macromolécules par calcul de la différence entre le temps d'impact et le temps reconstruit d'émission.

**39.** Spectromètre de masse selon l'une quelconque des revendications 1 à 38, caractérisé en ce que les portions de macromolécules (75) se trouvent sur une matrice (74) d'échantillons bidimensionnels.

**40.** Spectromètre de masse selon l'une quelconque des revendications 1 à 39, caractérisé par la détermination de la masse de fragments de DNA de séquences de DNA.

**41.** Spectromètre de masse selon l'une quelconque des revendications 1 à 39, caractérisé par la détermination de la masse de protéines ou de fragments de protéines de séquences de protéines.

**42.** Spectromètre de masse selon l'une quelconque des revendications 1 à 39, caractérisé par la détermination de la masse de protéines ou de fragments de protéines pour l'identification de protéines.

**43.** Spectromètre de masse selon l'une quelconque des revendications 1 à 39, caractérisé par la détermination de la masse de polymères ou de fragments de polymères pour l'identification de polymères.

FIG.1

FIG.2

FIG.3

FIG.4

FIG.5

FIG.6

EP 0 774 160 B1

FIG.7

FIG.8

FIG.9

**FIG.10**

FIG.11

FIG.12

FIG.13

FIG.14

FIG.15

FIG.16

FIG.17

FIG.18

FIG.19

FIG.20

FIG.21

FIG.22

FIG.23

FIG.24

FIG.25

EP 0 774 160 B1